(19) Europäisches Patentamt
European Patent Office
Office européen des brevets

(11) Numéro de publication: **0 283 390 B1**

(12) # FASCICULE DE BREVET EUROPEEN

(45) Date de publication de fascicule du brevet: **03.03.93**

(51) Int. Cl.[5]: **C07D 277/42**, C07D 277/84, C07D 277/60, C07D 417/12, A61K 31/425

(21) Numéro de dépôt: **88400572.9**

(22) Date de dépôt: **11.03.88**

(54) **Dérivés du thiazole actifs sur le système cholinergique, procédé d'obtention et compositions pharmaceutiques en contenant.**

(30) Priorité: **12.03.87 FR 8703398**

(43) Date de publication de la demande:
**21.09.88 Bulletin 88/38**

(45) Mention de la délivrance du brevet:
**03.03.93 Bulletin 93/09**

(84) Etats contractants désignés:
**AT BE CH DE ES FR GB GR IT LI LU NL SE**

(56) Documents cités:
**EP-A- 0 075 140**
**EP-A- 0 097 013**
**EP-A- 0 202 157**
**FR-A- 2 092 714**
**GB-A- 2 177 395**
**US-A- 2 975 182**
**US-A- 4 064 244**

**J. PHARM. SOC. JAPAN, vol. 72, 1952, pages 1009-1013, S. Saijo**

**J. ORG. CHEM., vol. 16, 1952, pages 225-231, H.H. Fox et al.**

**J. AM. CHEM. SOC., vol. 74, 1952, pages 2271-2273 et pages 2921-2922, I.A. Kaye et al.**

(73) Titulaire: **ELF SANOFI**
**32-34, rue Marbeuf**
**F-75008 Paris(FR)**

(72) Inventeur: **Biziere, Kathleen**
**60, rue de la Couronne**
**F-68400 Riedisheim(FR)**
Inventeur: **Olliero, Dominique**
**12, Allée Jeanne Bourgeois/Hameau de l'Aiguelongue**
**F-34100 Montpellier(FR)**
Inventeur: **Worms, Paul**
**10, rue du Devois**
**F-34980 Saint Gely Du Fesc(FR)**

(74) Mandataire: **Gillard, Marie-Louise et al**
**Cabinet Beau de Loménie 55, Rue d'Amsterdam**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

## Description

La présente invention concerne de nouveaux dérivés du thiazole, leur procédé de préparation et leur application en thérapeutique.

Selon un premier aspect, l'invention a pour objet en tant que produits nouveaux des dérivés du thiazole répondant à la formule générale :

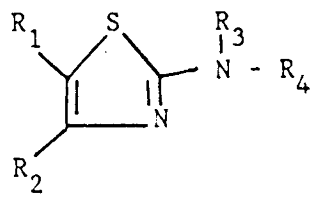

(I)

dans laquelle :

- $R_1$ et $R_2$ représentent chacun indépendamment l'hydrogène ; un groupe alkyle en $C_1$-$C_4$ ; un groupe phényle ; un groupe phényle substitué une ou plusieurs fois par un atome d'halogène, ou par un groupe alkyle en $C_1$-$C_4$, ou par un groupe alcoxy en $C_1$-$C_4$, un groupe nitro, ou un groupe hydroxy ; ou l'un des groupes $R_1$ et $R_2$ désigne l'hydrogène et l'autre représente : un groupe naphtyle ; un groupe benzyle ; un groupe $\alpha\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

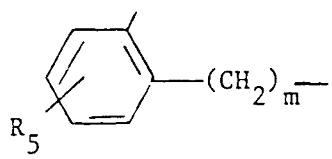

  où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ; avec la condition que si l'un des groupes $R_1$ ou $R_2$ désigne l'hydrogène l'autre est différent de H ou méthyle,
- $R_3$ représente l'hydrogène ou un groupe alkyle en $C_1$-$C_4$
- $R_4$ représente :
- un groupe

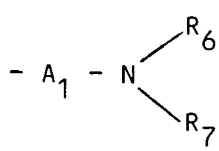

  dans lequel : $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$, ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine ;
- un groupe

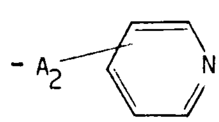

  où $A_2$ désigne un groupe $(CH_2)_m$ avec m = 0, 1, 2, 3 qui substitue le cycle pyridine en position 2, 3

ou 4 ;

- un groupe

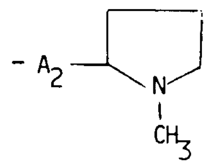

où $A_2$ est comme indiqué ci-dessus

- un groupe

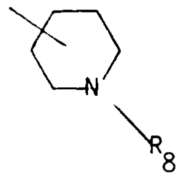

où $R_8$ désigne un groupe alkyle en $C_1$-$C_4$ ou encore le substituant

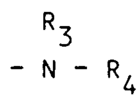

représente un groupe

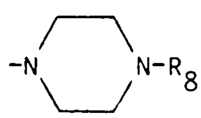

où $R_8$ est comme indiqué ci-dessus, à la condition dans ce cas, que si $R_1$ représente l'hydrogène ou un groupe alkyle en $C_1$-$C_4$, $R_2$ est autre qu'un groupe alkyle en $C_1$-$C_4$ ou qu'un groupe cyclohexyle, ainsi que leurs sels d'addition avec les acides minéraux ou organiques.

Le groupe phényle est de préférence substitué une ou plusieurs fois par un atome de chlore ou de fluor ou par un groupe méthyle.

Lorsque le substituant $R_4$ comprend un atome de carbone asymétrique, les composés (I) existent sous la forme de deux isomères optiques. Ces isomères optiques font partie intégrante de l'invention.

On notera que les composés de formule (I) dans laquelle $R_1$ et $R_2$ sont l'hydrogène, sont exclus de la définition ci-dessus, compte tenu des enseignements de SHIGEYA SAIJO publié dans J. PHARM. Soc. Japan, 1952, 72, 1009-1013, qui décrit des amino-2 thiazoles non substitués en positions 4 et 5, ayant une activité antihistaminique.

Les relations entre la structure et l'activité anti-histaminique de ces amino-2 thiazoles ont été rapportées dans l'article de H. H. Fox et al. J. ORG. CHEM., 1951, 16, 225-231. Leur préparation est décrite par I. A. KAYE et al. J. AM. CHEM. SOC., 1452, 74, 2271-2273 et 2921-2922.

Les composés de formule (I) dans laquelle l'un des groupes $R_1$ ou $R_2$ est l'hydrogène et l'autre est un groupe méthyle sont exclus de la définition des composés de l'invention en raison d'une non-faisabilité chimique.

Les composés de formule (I) dans laquelle $-NR_3R_4$ représente le groupe

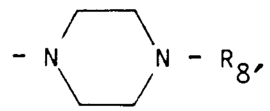

$R_1$ représente l'hydrogène ou un groupe alkyle en $C_1$-$C_4$ et $R_2$ est un groupe alkyle en $C_1$-$C_4$ ou un cyclohexyle sont décrits dans le brevet US 4 064 244 à titre d'agents anorexigènes, d'agents antimigraine ou d'agents pour augmenter la vigilance.

Selon un second aspect, l'invention a pour objet un procédé de préparation des composés de formule (I).

Le procédé selon l'invention, pour l'obtention des dérivés du thiazole définis ci-dessus, consiste à chauffer en milieu acide de pH 1 à 6 une thiourée substituée de formule :

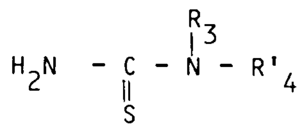

où $R_3$ a la signification indiquée ci-dessus et $R'_4$ a les mêmes significations que $R_4$, sauf dans les cas où $R_4$ contient une amine primaire ou secondaire où $R'_4$ désigne alors le groupe correspondant à $R_4$, dans lequel un hydrogène de ladite fonction amine a été remplacé par un groupe protecteur qui résiste à l'hydrolyse en milieu alcalin, avec un dérivé carbonylé alphabromé de formule :

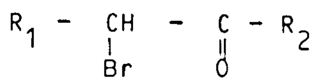

ou avec le dérivé bromé correspondant dans lequel le groupe carbonyle est protégé sous la forme d'acétal, pour obtenir les composés (I) et éventuellement à salifier le composé I selon un procédé connu.

La thiourée substituée mise en oeuvre dans le procédé de l'invention peut être obtenue par des procédés classiques, par exemple par action sur une amine primaire 1 d'isothiocyanate de benzoyle ou de pivaloylisothiocyanate.

Lorsque $R_3$ est l'hydrogène, le procédé de l'invention peut être représenté par le schéma réactionnel suivant :

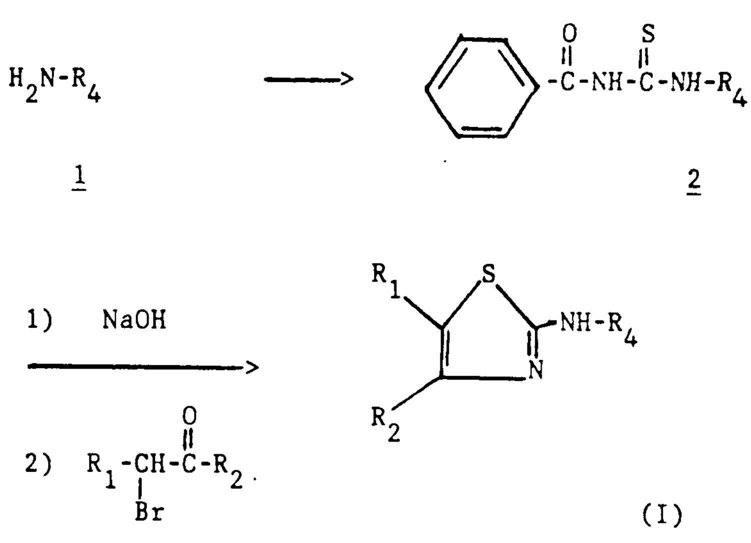


1 2 3 (I)

Par action sur l'amine primaire 1 d'un isothiocyanate de benzoyle, on obtient la benzoylthiourée substituée 2

En pratique, l'isothiocyanate de benzoyle est formé in situ par action du chlorure de benzoyle sur le thiocyanate de potassium au sein d'un solvant tel que l'acétone anhydre.

A la solution d'isothiocyanate de benzoyle ainsi obtenue, on ajoute une solution du dérivé aminé 1 dans un solvant convenable notamment le chlorure de méthylène et on chauffe à une température comprise entre 50 ° C et 100 ° C pendant 1/2 à 3 heures.

Après élimination des solvants, le mélange réactionnel est repris dans l'eau et le produit 2 est isolé de façon habituelle soit par extraction dans un solvant et purification, soit par chromatographie, soit par cristallisation directe.

A partir de la benzoylthiourée on obtient le produit (I) en 2 étapes.

Tout d'abord par chauffage du composé 2 avec une solution aqueuse diluée de soude, on élimine le groupe benzoyle. On acidifie le mélange réactionnel par addition d'un acide, par exemple d'acide chlorhydrique concentré jusqu'à un pH compris entre 1 et 6, puis on ajoute la solution du dérivé bromé 3 dans un solvant miscible à l'eau et de préférence un alcool.

On chauffe pendant quelques heures à une température comprise entre 70 et 100 ° C.

Après évaporation des solvants, on reprend le mélange réactionnel dans une solution de bicarbonate ou de carbonate de sodium et on isole le produit (I) par extraction par un solvant, tel que le chlorure de méthylène.

Après purification par les méthodes habituelles, le composé (I) est éventuellement transformé en sel par action d'un acide selon un procédé connu.

Dans ce procédé lorsque le dérivé bromé 3 est peu stable ou difficilement accessible, il peut être remplacé par le diméthylacétal correspondant.

Enfin, si $R_4$ comprend un groupe amine primaire ou secondaire, il est nécessaire de le bloquer pour éviter une réaction parasite avec le chlorure de benzoyle. On peut utiliser un groupe protecteur quelconque qui résiste à l'hydrolyse en milieu alcalin, tel que par exemple le groupe tertiobutoxycarbonyle (Boc).

A partir de l'amine $R_4NH_2$ ainsi protégé on obtient la benzoylthiourée 2 correspondante.
Par action de la soude diluée, on élimine le groupe benzoyle et directement par action du dérivé bromé 3 en pH acide on provoque la formation du cycle thiazole et l'élimination du groupe Boc conduisant aux composés (I) où $R_4$ comprend un groupe amine primaire ou secondaire.

Les produits de départ 1 et 3 sont des composés connus ou peuvent être préparés par des procédés connus. Pour les produits bromés 3 on pourra notamment se référer à l'ouvrage de HOUBEN WEYL "Methoden der Organischen Chemie" 4e éd. vol. 5/4 p. 171-188 - Georg Thieme Verlag.

Dans le cas particulier où $R_4$ représente un groupe

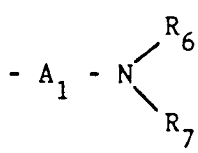

dans lequel au moins l'un des substituants $R_6$ et $R_7$ est l'hydrogène, les dérivés aminés 1 protégés peuvent être préparés à partir de l'aminonitrile $R_7NH-A'_1-CN$ (4). Celui-ci, traité par l'anhydride $(Boc)_2O$, conduit à l'aminonitrile

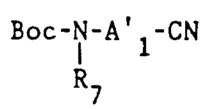

qui par réduction conduit à l'amine primaire correspondante

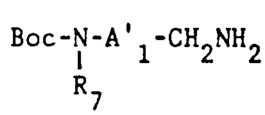

où A'$_1$ représente $A_1$ privé d'un groupe méthylène.

Dans tous les cas, et particulièrement lorsque le substituant $R_3$ est autre que l'hydrogène on peut préparer les composés (I) à partir du pivaloylisothiocyanate selon le schéma

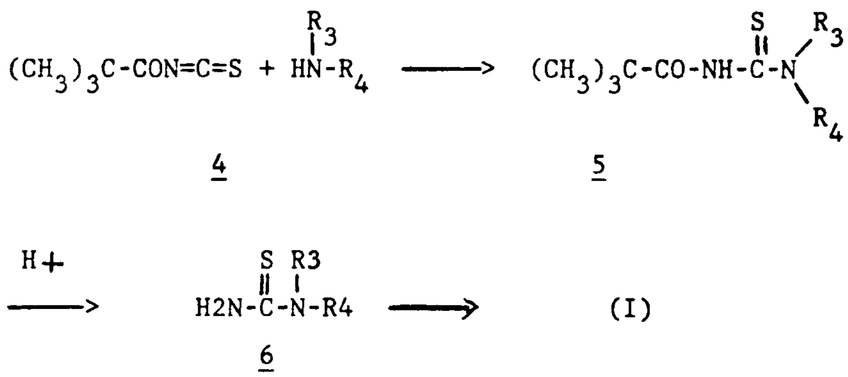

Par action sur l'amine 4 de pivaloylisothiocyanate on obtient la pivaloylthiourée 5.

En pratique, le pivaloylisothiocyanate est formé in situ par action du chlorure de pivaloyle sur un thiocyanate alcalin au sein d'un solvant anhydre tel que l'acétone et à température modérée (0 à 10 °C). On ajoute ensuite l'amine 4 et laisse réagir quelques heures à la même température.

Par hydrolyse à chaud en milieu acide fort le composé 5 est déacylé et conduit à la thiourée 6

Celle-ci est transformée en composé (I) par action d'un dérivé bromé 3 ainsi qu'indiqué précédemment.

Les exemples suivants non limitatifs permettront de mieux comprendre l'invention.

EXEMPLE 1 :

[(Diéthylamino-2 éthyl) amino]-2 phényl-5 thiazole, dichlorhydrate (SR 44318 A).

A) N-benzoyl N'-(diéthylaminoéthyl) thiourée.

A une suspension de 51 g de thiocyanate de potassium dans 300 ml d'acétone anhydre, on ajoute goutte à goutte à température ambiante la solution de 70 ml de chlorure de benzoyle dans 100 ml d'acétone anhydre. Après la fin de l'addition, le mélange réactionnel est chauffé au reflux pendant 5 minutes. A la solution chaude ainsi obtenue, on ajoute lentement sous forte agitation le solution de 85 ml de diéthylamino-2 éthylamine dans 100 ml de chlorure de méthylène de façon à maintenir le mélange au reflux. Après la fin de l'addition, on agite 1 heure à température ambiante puis on évapore les solvants et reprend le résidu dans l'eau glacée. On extrait 2 fois avec 200 ml de chlorure de méthylène et sèche la solution sur sulfate de magnésium. On évapore le solvant à siccité et le produit huileux obtenu est purifié par chromatographie sur colonne de silice. En éluant avec le mélange chlorure de méthylène-acétate d'éthyle 80-20 (vol/vol), on élimine des impuretés peu polaires. En éluant ensuite avec le mélange chlorure de méthylène-méthanol 95-5 (vol/vol), on obtient le produit attendu.

Poids : 78 g ; F : 54-56 °C.

B) SR 44 318 A.

On chauffe au reflux sous atmosphère d'azote pendant 30 minutes 3,5 g du composé préparé ci-dessus dans 18 ml d'une solution de soude 2,5 N. Aprés refroidissement on ajoute de l'acide chlorhydrique concentré jusqu'à un pH de 6 environ. On ajoute la solution de 2,48 g d'alpha bromo phényl acétaldéhyde dans 25 ml d'éthanol à 95° et chauffe au reflux pendant 1 heure sous atmosphère d'azote.

On évapore sous vide et ajoute une solution aqueuse à 10 % de carbonate de sodium. On extrait 2 fois avec du chlorure de méthylène et sèche la solution sur sulfate de magnésium. On évapore le solvant et chromatographie le résidu sur une colonne de silice en éluant avec le mélange chlorure de méthylène/méthanol 95/5 (vol/vol). On obtient une huile (1,46 g).

On dissout cette huile dans l'éther anhydre et ajoute une solution de gaz chlorhydrique dans l'éther anhydre et laisse cristalliser. On essore les cristaux qu'on lave avec de l'éther anhydre et sèche sous vide.

F : 178 °C.

EXEMPLES 2 A 108 :

A) En opérant comme dans l'exemple 1-A mais en faisant varier les composés aminés utilisés, on obtient les thiourées substituées rassemblées dans le tableau 1 ci-après.

TABLEAU 1

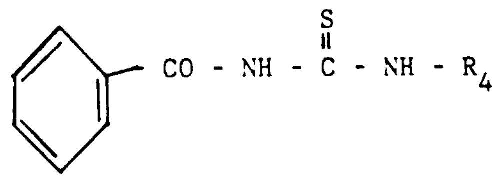

| $R_4$ | Constante physique |
|---|---|
| $-(CH_2)_2$ - 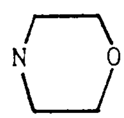 | F = 119 - 120°C |
| $-(CH_2)_3$ - 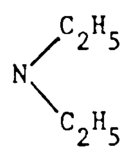 | Huile, CCM : Rf = 0,51 $CH_2Cl_2$-MeOH 90/10 vol/vol |
| $-(CH_2)_3$ - 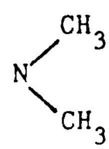 | F = 67 - 69°C |
| $-(CH_2)_3$ - 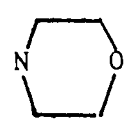 | Huile |
| $-(CH_2)_2$ - 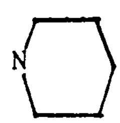 | F = 98 - 99°C |
| $-(CH_2)_2$ - 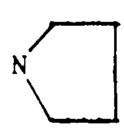 | F = 70 - 71°C |

| | |
|---|---|
| 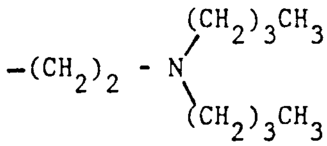 | F = 48-49°C |
| 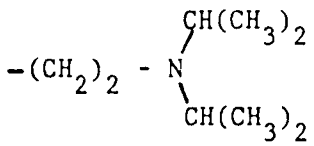 | F = 93 - 94°C |
| 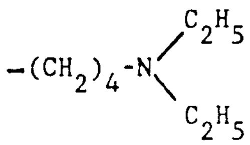 | Huile |
| 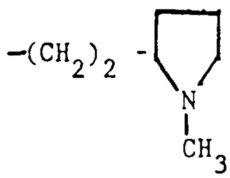 | F = 65 - 67°C |
| 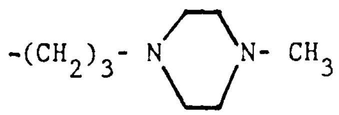 | F = 55 - 57°C |
| 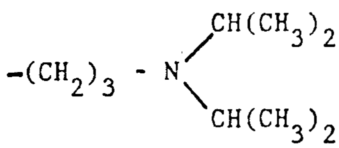 | Huile |
| 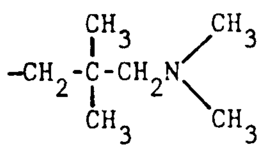 | F = 57 - 58°C |
| 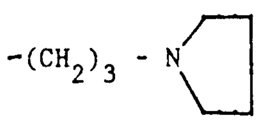 | F = 73 - 75°C |
| 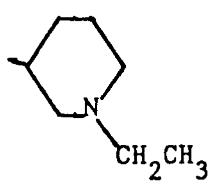 | Huile |
| 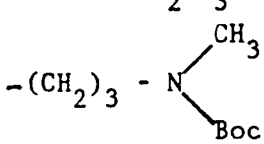 | F = 72 - 74°C |

| | |
|---|---|
| $-(CH_2)_2$ - 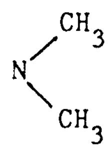 | F = 74 - 75°C |
| $-(CH_2)_4$ - 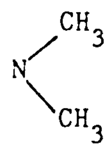 | F = 31 - 35°C |
| $-(CH_2)_3$ - 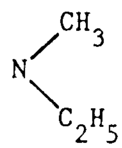 | Huile |
| $-(CH_2)_3$ - 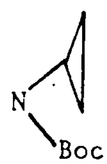 | F = 45 - 48°C |
| $-CH_2$ - 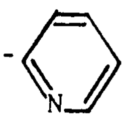 | F = 152 - 153°C |
| $-CH_2$- 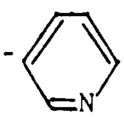 | F = 98 - 100°C |
| $-CH_2$ - 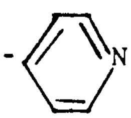 | F = 158 - 159°C |
| $-(CH_2)_2$ - 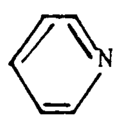 | F = 146 - 147°C |
| $-(CH_2)_2$ - 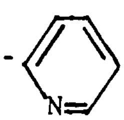 | F = 118 - 119°C |
| $-(CH_2)_2$ - 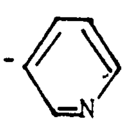 | F = 142 - 143°C |

| | |
|---|---|
| $-(CH_2)_3$ - 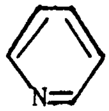 | F = 81 - 82°C |
| $-(CH_2)_5$ 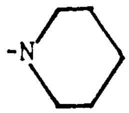 | F = 118 - 120°C |

B) En opérant comme dans l'exemple 1-B à partir de ces différentes thiourées et en faisant varier le dérivé oxohalogéné qui lui est opposé on obtient les produits de formule (I) rassemblés dans le tableau 2 ci-après.

TABLEAU 2

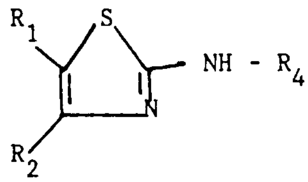

| Ex n° | N° de Code SR | $R_1$ | $R_2$ | $R_4$ | Sel F°C (solvant) |
|---|---|---|---|---|---|
| 2 | 44244 A | H | 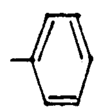 | $-(CH_2)_2-$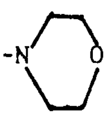 | Dichlorhydrate 220-221 (iPrOH-$Et_2O$) |
| 3 | 44284 A | " | " | $-(CH_2)_3-$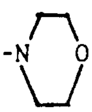 | Dichlorhydrate 216-217 (iPrOH) |
| 4 | 44285 A | 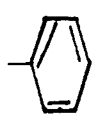 | H | $-(CH_2)_2-$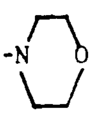 | Dichlorhydrate 264-265 (iPrOH-EtOH95) |
| 5 | 44286 A | H | 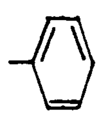 | $-(CH_2)_2-$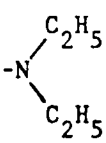 | Succinate 110-111 (iPrOH-$iPr_2O$) |
| 6 | 44345 A | " | " | $-(CH_2)_3-$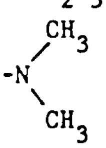 | Dichlorhydrate 181-182 ($Et_2O$) |
| 7 | 44346 A | 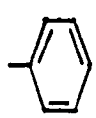 | H | $-(CH_2)_3-$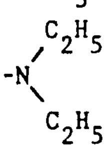 | Dichlorhydrate 0,5 H20 143-144 (acétone) |

| | | | | | |
|---|---|---|---|---|---|
| 8 | 44347 A | H | 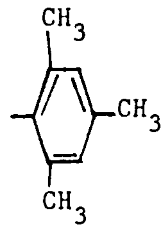 | $-(CH_2)_3$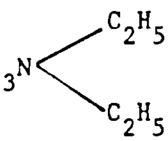 | Dichlorhydrate 199-200 (cyclohexane, $Et_2O$) |
| 9 | 44372 A | " | " | $-(CH_2)_2$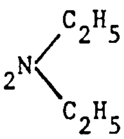 | Dichlorhydrate 1 $H_2O$ 147-148 (acétone, $Et_2O$) |
| 10 | 44373 A | 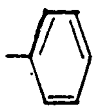 | $-CH_3$ | $-(CH_2)_3$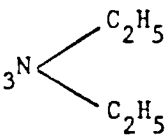 | Dichlorhydrate 0,5 $H_2O$ 184-5 (acétone, $Et_2O$) |
| 11 | 44374 A | H | 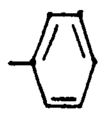 | " | Dimaléate 109-111 (iPrOH, $Et_2O$) |
| 12 | 44421 A | H | 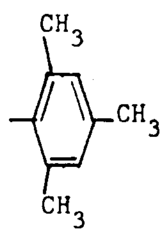 | $-(CH_2)_3$ -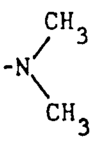 | Dichlorhydrate 224-226 (iPrOH, $Et_2O$) |
| 13 | 44422 A | H | $- CH_3$ | $-(CH_2)_2$-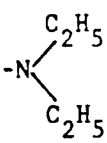 | Dichlorhydrate 162-163 (iPrOH, $Et_2O$) |
| 14 | 44424 A | H | 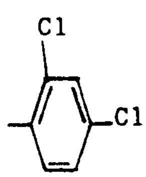 | $-(CH_2)_2$-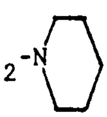 | Dichlorhydrate 216-217 (iPrOH, $Et_2O$) |
| 15 | 44434 A | $-CH_3$ | 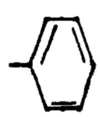 | $-(CH_2)_3$-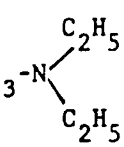 | Dichlorhydrate 0,5 H2O 148-149 (iPrOH, $Et_2O$) |
| 16 | 44435 A | 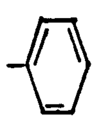 | 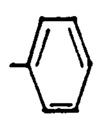 | $-(CH_2)_3$-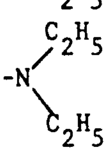 | Dichlorhydrate 0,5 H2O 167-168 (iPrOH, $iPr_2O$) |

| | | | | | |
|---|---|---|---|---|---|
| 17 | 44436 A | " | $-CH_3$ | 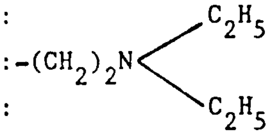 | Dichlorhydrate 0,5 H20 154-155 ($Et_2O$) |
| 18 | 44437 A | 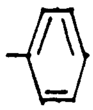 | 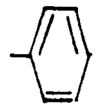 | 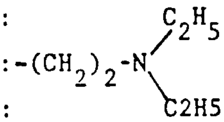 | Dichlorhydrate 193-194 ($Et_2O$) |
| 19 | 44438 A | " | H | 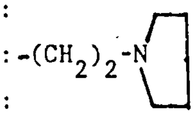 | Dichlorhydrate 0,5 $H_2O$ 190-195 (dec) ($Et_2O$) |
| 20 | 44451 A | H | 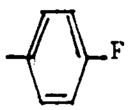 | 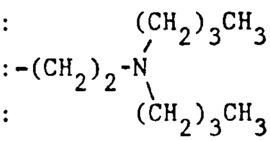 | Dichlorhydrate 141-142 ($Et_2O$, PrOH) |
| 21 | 44465 A | H | 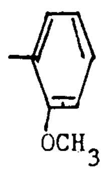 | 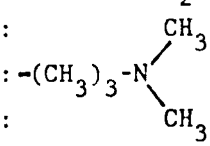 | Dichlorhydrate 184-186 (dec) (iPrOH, $Et_2O$) |
| 22 | 44466 A | 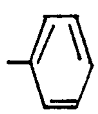 | $-CH_3$ | 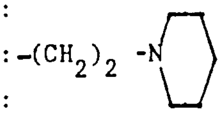 | Dichlorhydrate 204-207 (dec) (iPrOH) |
| 23 | 44467 A | 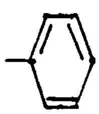 | $-CH_3$ | 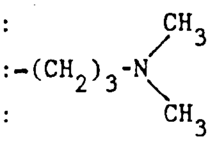 | Dichlorhydrate 235-236 (dec) (iPrOH, $iPr_2O$) |
| 24 | 44468 A | H | 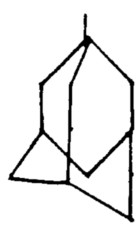 | 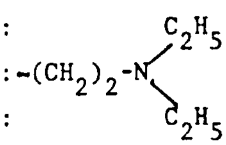 | Dichlorhydrate 196-199 (iPrOH, $iPr_2O$) |
| 25 | 44469 A | H | 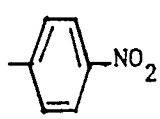 | 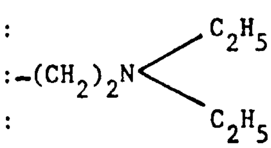 | Dichlorhydrate 185-190 (dec) (iPrOH, $Et_2O$) |

| | | | | | |
|---|---|---|---|---|---|
| 26 | 44488 A | 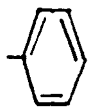 | $-CH_3$ | $-(CH_2)_2$ 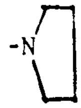 | Dichlorhydrate 178-180 ($Et_2O$) |
| 27 | 44489 A | " | " | $-(CH_2)_2$- 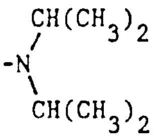 | Dichlorhydrate $1H_2O$ 130-135 (iPrOH, $iPr_2O$) |
| 28 | 44490 A | " | H | $-(CH_2)_2$ 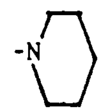 | Dichlorhydrate 250 (dec) ($Et_2O$) |
| 29 | 44491 A | " | H | $-(CH_2)_2$- 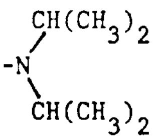 | Dichlorhydrate 1 $H_2O$ 93 ($Et_2O$) |
| 30 | 44492 A | " | H | $-(CH_2)_2$- 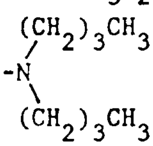 | Dichlorhydrate 147 (acétone, $Et_2O$) |
| 31 | 44493 A | H | 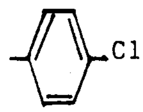 | $-(CH_2)_3$ 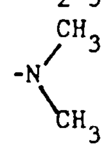 | Dichlorhydrate 1 $H_2O$ 141 (iPrOH, $iPr_2O$, $H_2O$) |
| 32 | 44494 A | H | 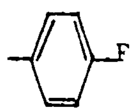 | $-(CH_2)_3$- 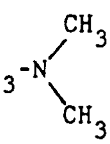 | Dichlorhydrate 1 $H_2O$ 114 (iPrOH, $Et_2O$) |
| 33 | 44515 A | 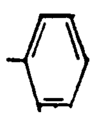 | 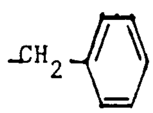 | $-(CH_2)_3$- 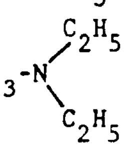 | Dichlorhydrate 1 $H_2O$ 88-89 ($Et_2O$) |
| 34 | 44516 A | H | 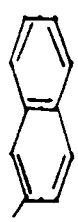 | $-(CH_2)_2$- 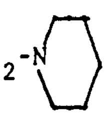 | Dichlorhydrate 1 $H_2O$ 91-93 ($Et_2O$) |

| | | | | | |
|---|---|---|---|---|---|
| 35 | 44573 A | H | 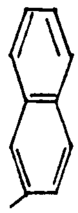 | 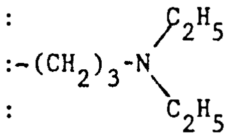 | Dichlorhydrate 96°C ($Et_2O$) |
| 36 | 44574 A | H | 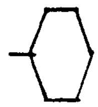 | 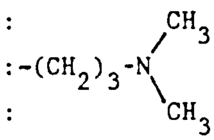 | Dichlorhydrate 155°C ($Et_2O$-iPrOH) |
| 37 | 44575 A | H | 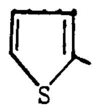 | 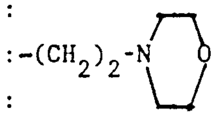 | Dichlorhydrate 182°C (EtOH95-iPrOH) |
| 38 | 44576 A | H | 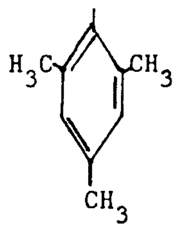 | 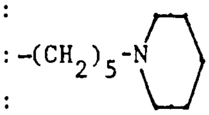 | Dichlorhydrate 150-155 ($Et_2O$) |
| 39 | 44749 A | H | 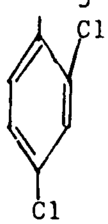 | 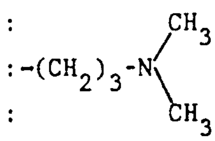 | Dichlorhydrate 194-196°C ($Et_2O$) |
| 40 | 44750 A | H | " | 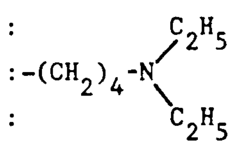 | Fumarate 121-3°C ($Et_2O$) |

| | | | | | |
|---|---|---|---|---|---|
| 41 | 44768 A | H | 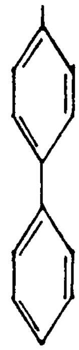 | 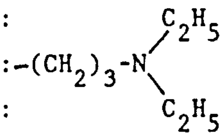 | Fumarate 182-4°C ($Et_2O$) |
| 42 | 44769 A | $CH_3$ | 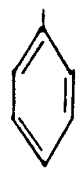 | 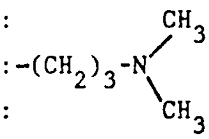 | Dichlorhydrate 191°C ($Et_2O$) |
| 43 | 44770 A | H | 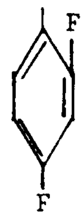 | " " | Dichlorhydrate ($1H_2O$) 168°C ($Et_2O$) |
| 44 | 44786 A | H | 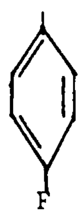 | 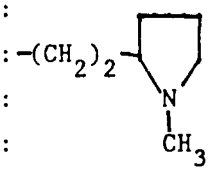 | Difumarate 138-140°C ($Et_2O$) |
| 45 | 44791 A | H | 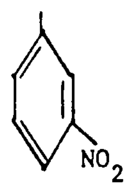 | 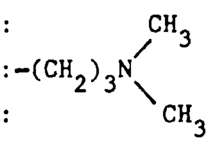 | Dichlorhydrate ($0,5H_2O$) 175-6°C ($Et_2O$) |
| 46 | 44814 A | H | 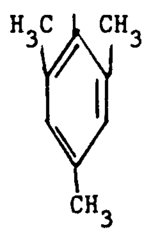 | 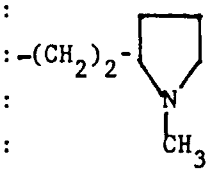 | Difumarate 140-4°C ($Et_2O$) |

| | | | | | |
|---|---|---|---|---|---|
| 47 | 44832 A | H | 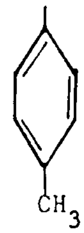 | 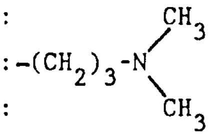 | Dichlorhydrate ($0,5H_2O$) 165°C ($Et_2O$) |
| 48 | 44862 A | H | 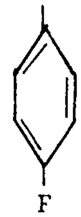 | 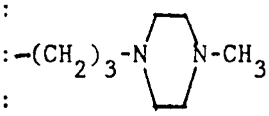 | Trichlorhydrate ($1H_2O$) 249-251°C ($Et_2O$) |
| 49 | 44887 A | H | 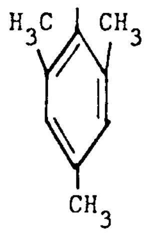 | 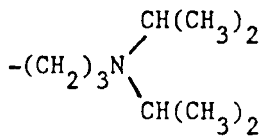 | Dichlorhydrate ($1,5H_2O$) 78-82°C ($Et_2O$) |
| 50 | 44907 A | H | " " | 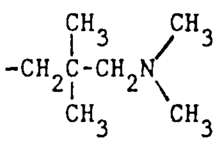 | Difumarate ($0,5H_2O$) 173-4°C ($Et_2O$) |
| 51 | 44908 A | H | " " | 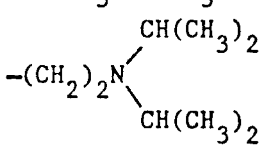 | Dichlorhydrate 114-6°C ($Et_2O$) |
| 52 | 44914 A | H | 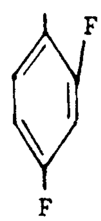 | 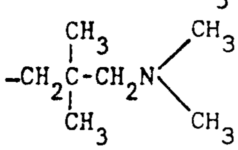 | Dichlorhydrate 180-5°C ($Et_2O$) |
| 53 | 44915 A | H | 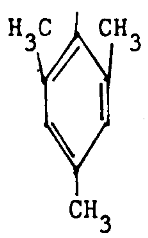 | 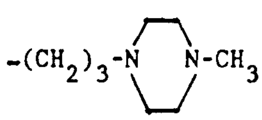 | Trichlorhydrate 145-150°C ($Et_2O$-iPrOH) |

| | | | | | |
|---|---|---|---|---|---|
| 54 | 44916 A | H | 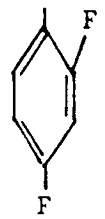 | 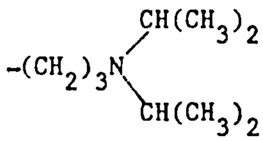 | Dichlorhydrate 165-7°C (iPrOH-$Et_2O$) |
| 55 | 44963 A | 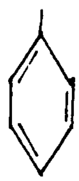 | $-C_2H_5$ | 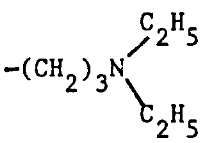 | Dichlorhydrate (1$H_2O$) 155-6°C (Acétone) |
| 56 | 44964 A | H | 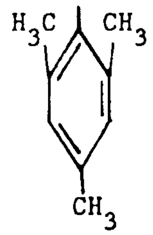 | 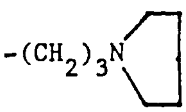 | Difumarate 147-8°C ($Et_2O$) |
| 57 | 44965 A | H | " " | 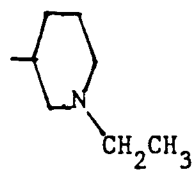 | Fumarate (1$H_2O$) 226-8°C ($Et_2O$) |
| 58 | 44967 A | 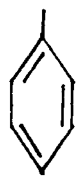 | H | 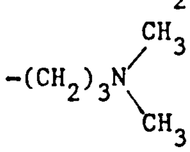 | Dichlorhydrate 82°C ($Et_2O$) |
| 59 | 45001 A | H | 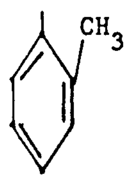 | 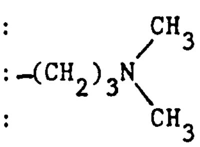 | Fumarate 123°C ($Et_2O$) |
| 60 | 45015 A | 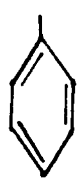 | 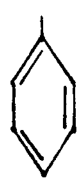 | " " | Dichlorhydrate (0,5$H_2O$) 210°C ($Et_2O$) |

| | | | | | |
|---|---|---|---|---|---|
| 61 | 45016 A | 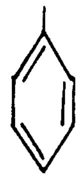 | 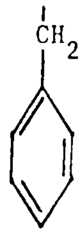 | " " | Dichlorhydrate ($0,5H_2O$) 207-8°C ($Et_2O$) |
| 62 | 45018 A | H | 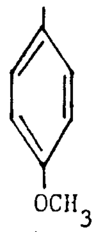 | " " | Dichlorhydrate ($0,5H_2O$) 165-7°C ($Et_2O$) |
| 63 | 45023 A | H | 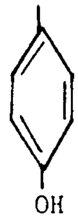 | " " | Dichlorhydrate 238-240°C ($Et_2O$) |
| 64 | 45030 A | H | 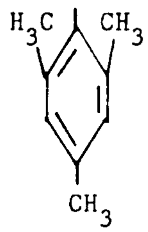 | $-(CH_2)_3NHCH_3$ | Difumarate 138-140°C |
| 65 | 45035 A | H | 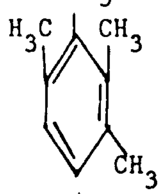 | 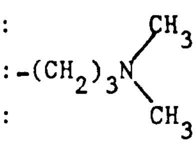 | Dichlorhydrate ($0,5H_2O$) 222-5°C ($Et_2O$) |
| 66 | 45038 A | H | 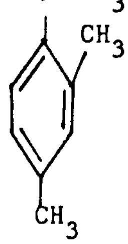 | " " | Trihémifumarate 129-130°C ($Et_2O$) |

| | | | | | |
|---|---|---|---|---|---|
| 67 | 45043 A | 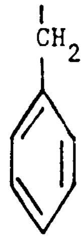 | H | " " | Trihémifumarate 102-3°C ($Et_2O$) |
| 68 | 45079 A | H | 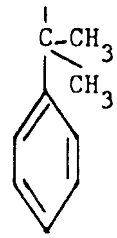 | " " | Dichlorhydrate ($1H_2O$) 130-2°C ($Et_2O$) |
| 69 | 45080 A | H | 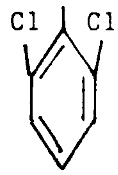 | " " | Dichlorhydrate ($0,5H_2O$) 228°C ($Et_2O$) |
| 70 | 45088 A | 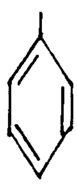 | $-C_2H_5$ | " " | Trihémifumarate 125-6°C ($Et_2O$) |
| 71 | 45107 A | H | 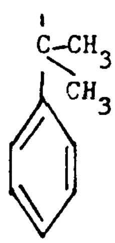 | 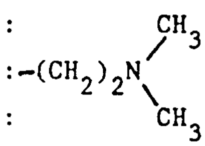 | Fumarate 114°C ($Et_2O$) |
| 72 | 45108 A | H | 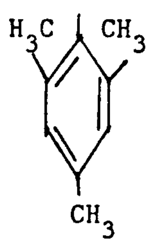 | 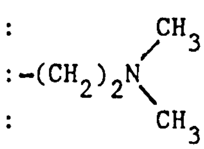 | Mono-chlorhydrate ($1H_2O$) 202°C ($Et_2O$) |
| 73 | 45125 A | H | " " | 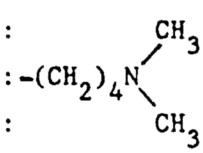 | Difumarate 132-3°C ($Et_2O$) |

| | | | | | |
|---|---|---|---|---|---|
| 74 | 45137 A | 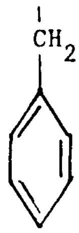 | H | 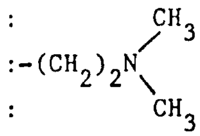 | Mono-chlorhydrate 183°C ($Et_2O$) |
| 75 | 45154 A | H | 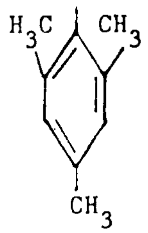 | 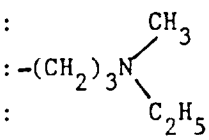 | Difumarate 126-7°C ($Et_2O$) |
| 76 | 45190 A | H | 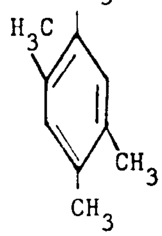 | 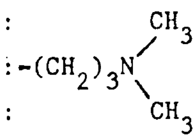 | Difumarate (0,5$H_2O$) 116-8°C ($Et_2O$) |
| 77 | 44813 A | H | 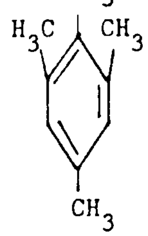 | 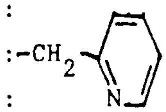 | Dichlorhydrate 246-8°C ($Et_2O$) |
| 78 | 44833 A | H | 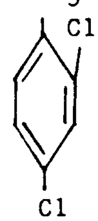 | " " | Dichlorhydrate 125-6°C ($Et_2O$) |
| 79 | 44839 A | H | 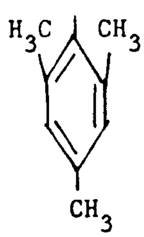 | 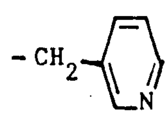 | Dichlorhydrate (0,5$H_2O$) 245°C ($Et_2O$) |

| | | | | | |
|---|---|---|---|---|---|
| 80 | 44840 A | H | 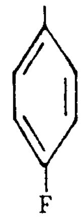 | 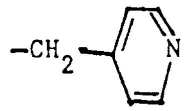 | Dichlorhydrate ($1H_2O$) 219-220°C ($Et_2O$) |
| 81 | 44856 A | H | 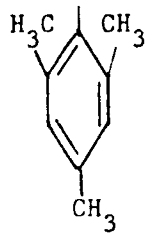 | 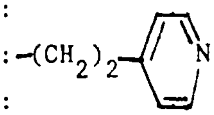 | Dichlorhydrate 225-6°C ($Et_2O$) |
| 82 | 44863 A | H | 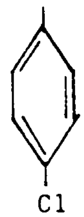 | 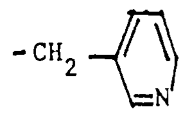 | Dichlorhydrate ($2H_2O$) 111-2°C ($Et_2O$) |
| 83 | 44864 A | H | 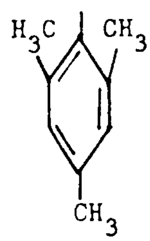 | 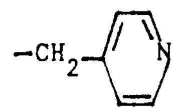 | Dichlorhydrate ($2H_2O$) 237-9°C ($Et_2O$) |
| 84 | 44902 A | 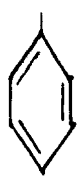 | 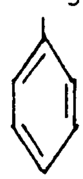 | 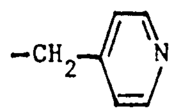 | Dichlorhydrate ($0,5H_2O$) 183°C ($Et_2O$) |
| 85 | 44903 A | H | 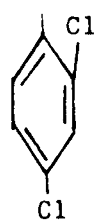 | 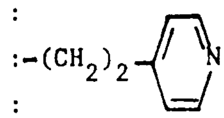 | Dichlorhydrate 200-2°C ($Et_2O$) |
| 86 | 44904 A | 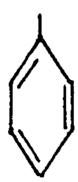 | 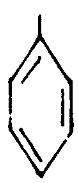 | 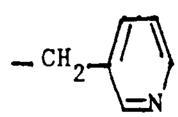 | Dichlorhydrate ($1H_2O$) 133-5°C ($Et_2O$) |

| | | | | | |
|---|---|---|---|---|---|
| 87 | 44905 A | H | 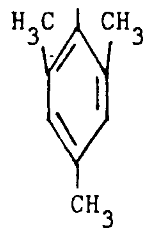 | 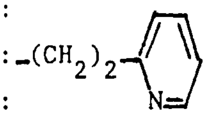 | Dichlorhydrate (1,5$H_2O$) 122°C ($Et_2O$) |
| 88 | 44906 A | 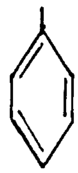 | 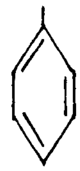 | 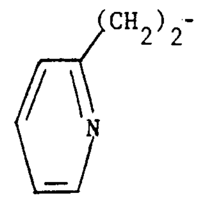 | Dichlorhydrate (1$H_2O$) 173°C ($Et_2O$) |
| 89 | 44913 A | H | 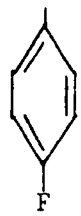 | 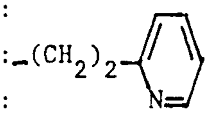 | Mono-chlorhydrate 171°C ($Et_2O$) |
| 90 | 44911 A | 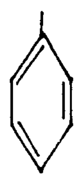 | 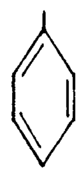 | 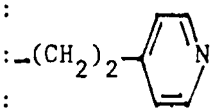 | Mono-chlorhydrate 221°C ($Et_2O$) |
| 91 | 44912 A | " " | " " | 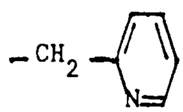 | Mono-chlorhydrate 150°C ($Et_2O$) |
| 92 | 44966 A | 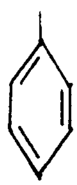 | H | 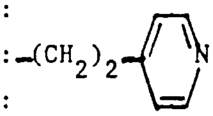 | Fumarate 148°C ($Et_2O$) |
| 93 | 44968 A | " " | H | 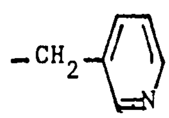 | Fumarate 147-8°C ($Et_2O$) |
| 94 | 44998 A | " " | H | 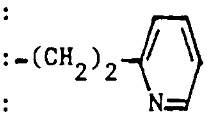 | Dichlorhydrate (1$H_2O$) 211°C ($Et_2O$) |

| | | | | | |
|---|---|---|---|---|---|
| 95 | 45002 A | " " | H | 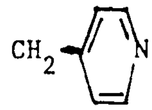 | Dichlorhydrate ($2H_2O$) 94°C ($Et_2O$) |
| 96 | 45106 A | H | 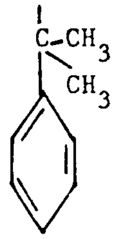 | 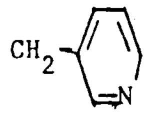 | Dichlorhydrate 137°C ($Et_2O$) |
| 97 | 45124 A | 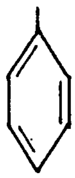 | $C_2H_5$ | 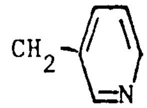 | Dichlorhydrate 213-6°C ($Et_2O$) |
| 98 | 45122 A | H | 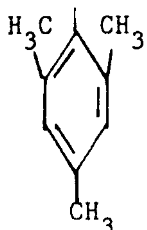 | 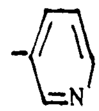 | Mono-chlorhydrate > 250°C ($Et_2O$) |
| 99 | 45123 A | H | 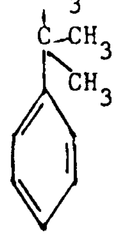 | " " | Mono-chlorhydrate 184°C ($Et_2O$) |
| 100 | 45169 A | H | 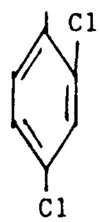 | 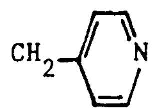 | Dichlorhydrate 201-4°C (iPrOH-EtOH) |
| 101 | 45185 A | 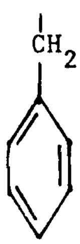 | H | 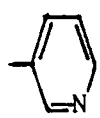 | Base ($1H_2O$) 163°C ($Et_2O$) |

| | | | | | |
|---|---|---|---|---|---|
| 102 | 45221 A | H | 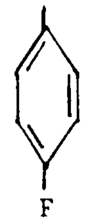 | $-(CH_2)_2-$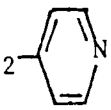 | Dichlorhydrate 207-9°C (iPrOH) |
| 103 | 45253 A | H | 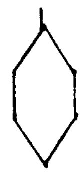 | $-CH_2-$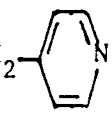 | Mono-chlorhydrate 160°C ($Et_2O$) |
| 104 | 45240 A | H | " " | $-CH_2-$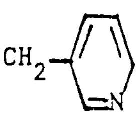 | Dichlorhydrate 191-2°C ($Et_2O$) |
| 105 | 45239 A | H | 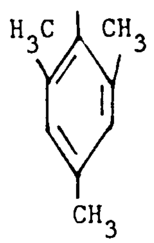 | $-(CH_2)_2-$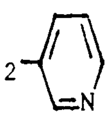 | Dichlorhydrate 227-9°C ($Et_2O$) |
| 106 | 45255 A | H | 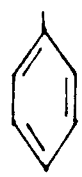 | $-CH_2-$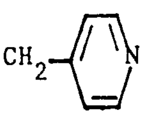 | Mono-chlorhydrate ($0,5H_2O$) 200-1°C ($Et_2O$) |
| 107 | 45264 A | H | 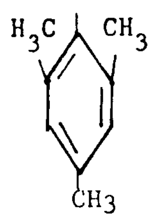 | $-(CH_2)_3-$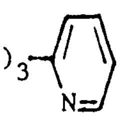 | Dichlorhydrate 221-3°C (iPrOH-$Et_2O$) |
| 108 | 45258 A | H | " | $(CH_2)_3-NH-$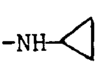 | oxalate 170-172°C (acétone) |

iPrOH = Isopropanol ; PrOH = Propanol ; EtOH 95 = Ethanol 95

$Et_2O$ = Ether éthylique anhydre ; $iPr_2O$ = Ether isopropylique

EXEMPLE 109 :

[(Amino-3 propyl) amino]-2 méthyl-5 phényl-4 thiazole, dichlorhydrate (SR 44 514 A).

A) N-Boc- amino-3 propionitrile.

A la solution de 26 g de fumarate d'amino-3 propionitrile dans 100 ml d'eau, on ajoute 56 ml de triéthylamine puis on chauffe à 50 °C. On ajoute alors sous forte agitation 46 g d'acide dicarbonique bis tertiobutylester ($Boc_2O$) en solution dans 100 ml de dioxanne.

Après évaporation du solvant, on reprend par du dichlorométhane. On lave la solution organique avec une solution aqueuse de carbonate de sodium à 5 % puis avec du tampon sulfate pH 2. On sèche la solution sur sulfate de magnésium et évapore le solvant. Le résidu (38,8 g) cristallise.

B) N-Boc-amino-3 propylamine.

On dissout 20 g du produit précédent dans le mélange de 400 ml d'eau, 40 ml d'ammoniaque et 50 ml d'éthanol. On ajoute du Nickel de Raney et hydrogène à température et pression ordinaires.

Après filtration du catalyseur, on évapore les solvants sous vide. On reprend le résidu dans l'eau salée et extrait avec de l'acétate d'éthyle. On sèche la solution sur sulfate de magnésium et évapore le solvant. On obtient 12,6 g du produit attendu.

C) N-benzoyl N'-(N-Boc amino-3 propyl) thiourée.

A la suspension de 7,05 g de thiocyanate de potassium dans 50 ml d'acétone anhydre, on ajoute goutte à goutte la solution de 6,38 ml de chlorure de benzoyle dans 20 ml d'acétone anhydre puis on chauffe au reflux pendant 5 minutes.

A la solution chaude, on ajoute lentement sous vive agitation la solution de 12,6 g de N-Boc amino-3 propylamine dans 20 ml de chlorure de méthylène.

Après la fin de l'addition, on laisse 1 heure sous agitation puis on évapore les solvants et reprend le résidu dans l'eau glacée. On extrait avec de l'acétate d'éthyle et sèche la solution sur sulfate de magnésium. On évapore le solvant et reprend le résidu dans 100 ml d'éther anhydre froid.

Il se forme des cristaux qu'on essore et lave avec de l'éther froid et sèche à l'étuve à 70 ° C.

Poids : 8,3 g ; F : 104-105 ° C.

D) SR 44 514 A.

On chauffe au reflux pendant 15 minutes, 3,37 g du produit obtenu ci-dessus dans 14 ml d'une solution de soude 2,5 N.

Après refroidessement, on ajoute de l'acide chlorhydrique concentré jusqu'à pH = 1. On ajoute 2,13 g de bromo-2 propiophénone dissous dans 20 ml d'éthanol et chauffe au reflux pendant 1 heure sous atmosphère d'azote.

On traite le mélange comme indiqué dans l'exemple 1-B et obtient une huile.

On forme le dichlorhydrate en solution dans le méthanol par addition d'éther chlorhydrique.

F : 100-110 ° C

EXEMPLES 110 ET 111 :

A partir de la thiourée substitueé préparée à l'exemple 109 C et en opérant comme dans l'exemple 109 D mais en faisant varier le dérivé bromé utilisé, on obtient de la même façon :

EXEMPLE 110 / (Amino-3 propyl)amino /-2 (fluoro-4 phényl)-4 thiazole (SR 44886 A) isolé sous forme de fumarate ($0,5H_2O$). F : 162-4 ° C.

EXEMPLE 111 / (Amino-3 propyl) amino /-2 (triméthyl-2,4,6 phényl)-4 thiazole (SR 44949 A) isolé sous forme de fumarate. F : 163-4 ° C.

EXEMPLE 112 [N-méthyl N-(pyridyl-3 méthyl) amino] -2 (triméthyl-2,4,6 phényl)-4 thiazole, oxalate (SR 45206 A)

(I) $R_1$ = H $R_2$ = 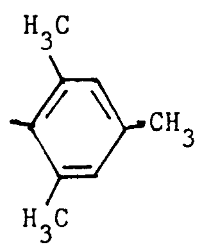 $R_3$ = $CH_3$ $R_4$ = $-CH_2$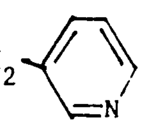

A) N-méthyl N-(pyridyl-3 méthyl) thiourée

A une suspension de 16,2 g de thiocyanate de sodium dans 90 ml d'acétone anhydre, refroidie à + 4° C, on ajoute goutte à goutte sous bonne agitation 24,6 ml de chlorure de pivaloyle. On laisse sous agitation pendant 3 heures à + 4° C puis on ajoute goutte à goutte 25 g de méthylaminométhyl-3 pyridine de façon à maintenir la température entre 0 et + 4° C. On laisse remonter la température à 20° C et agite pendant 15 heures à cette température. On évapore le mélange sous vide et reprend le résidu dans 100 ml d'acide chlorhydrique concentré. On chauffe 1 heure à 95° C. Après refroidissement on extrait 2 fois avec du chlorure de méthylène puis on alcalinise la phase aqueuse par une solution de soude concentrée. On extrait 4 fois avec 200 ml de chlorure de méthylène. On réunit les extraits organiques et sèche sur sulfate de magnésium. On évapore le solvant et le résidu cristallise. On triture les cristaux avec 100 ml d'éther et essore. Après séchage, on obtient 26,7 g du composé attendu. F : 119°-120° C.

B) SR 45206 A

On chauffe au reflux pendant 4 heures le mélange de 3,6 g de la thiourée préparée ci-dessus, 6 g de triméthyl-2,4,6 bromure de phénacyle, 20 ml d'eau, 50 ml d'éthanol et 2 ml d'acide chlorhydrique concentré.

Après évaporation sous vide, on reprend le résidu dans 150 ml de chlorure de méthylène et on extrait avec 150 ml d'une solution aqueuse d'acide chlorhydrique 1 N. On réextrait la phase organique 3 fois avec 100 ml d'acide chlorhydrique 1 N et réunit les extraits acides. On alcanilise par de la soude et extrait 4 fois avec 150 ml de chlorure de méthylène. On réunit les extraits organiques, sèche sur sulfate de magnésium et évapore le solvant. Le résidu huileux est chromatographié sur colonne de silice (100 g). En éluant avec un mélange chlorure de méthylène-méthanol 97-3 vol/vol., on obtient 4,5 g de produit huileux.

OXALATE

On dissout les 4,5 g de produit ci-dessus dans 30 ml d'acétone et ajoute la solution de 1,4 g d'acide oxalique dans 40 ml d'acétone.

Il se forme des cristaux jaune clair qu'on essore, lave avec un peu d'acétone et sèche.

On obtient 4,3 g du produit attendu. F : 160-1° C.

EXEMPLES 113 A 117

En opérant comme dans l'exemple 112 A, on obtient de la même façon les thiourées substituées réunies dans le tableau 3.

TABLEAU 3

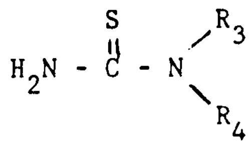

| 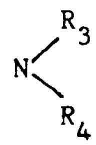 | Point de fusion |
|---|---|
| 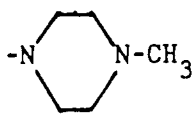 | F : 174-5°C |
| 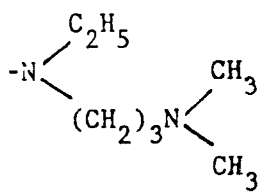 | F : 40-4°C |
| 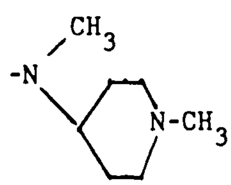 | F : 141-3°C |

B) A partir de ces différentes thiourées, en opérant comme dans l'exemple 112 b, on obtient les composés I du tableau 4.

TABLEAU 4

| N° Ex. | N° Code SR | $R_1$ | $R_2$ | $N(R_3)(R_4)$ | Sel isolé Point de fusion °C |
|---|---|---|---|---|---|
| 113 | 45215 A | H | 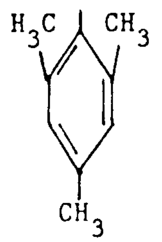 | 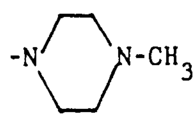 | Mono-chlorhydrate ($1H_2O$) 145-7°C ($Et_2O$) |
| 114 | 45216 A | 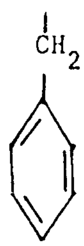 | H | " " | Mono-chlorhydrate 210-1°C ($Et_2O$) |
| 115 | 45223 A | H | 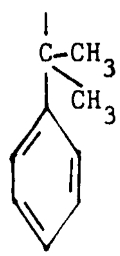 | " " | Dichlorhydrate 231-2°C ($Et_2O$) |
| 116 | 45191 A | H | 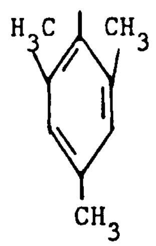 | 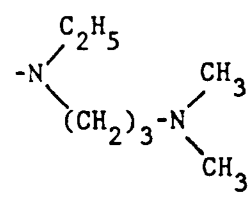 | Oxalate 44-5°C |

| | | | | | |
|---|---|---|---|---|---|
| 117 | 45257 A | H | " " | 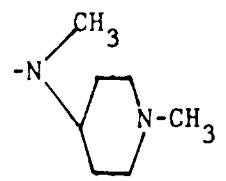 | Dichlorhydrate 224-6°C ($iPrOH-iPr_2O$) |

EXEMPLE 118

(Morpholino-2 éthylamino)-2 dihydro-4,5 naphto [1,2-d] thiazole, dichlorhydrate (SR 44 273 A).

On chauffe au reflux sous atmosphère d'azote pendant 15 minutes, 3,66 g de N-benzoyl N'-(morpholinoéthyl) thiourée dans 17,5 ml d'une solution de soude 2,5 N. Après refroidissement, on ajoute de l'acide chlorhydrique concentré jusqu'à pH 7. On ajoute la solution de 3 g de bromo-2 tétrahydro-1,2,3,4 naphtalènone-1 dans 20 ml d'éthanol et chauffe au reflux pendant 1 heure. Après évaporation du solvant, on neutralise par addition de bicarbonate de sodium et extrait 2 fois avec du dichlorométhane. On sèche la phase organique sur sulfate de magnésium et chromatographie sur colonne de silice. On élue avec un mélange dichlorométhane-méthanol (95-5 ; vol/vol).

Après évaporation des solvants, on reprend le résidu huileux dans l'éther et précipite le chlorhydrate dans l'éther. On essore et recristallise dans un mélange isopropanol-éthanol à 95.

F : 255-257 ° C.

EXEMPLES 119 A 124 :

En opérant comme dans l'exemple 118 mais en faisant varier les dérivés bromés et/ou les benzoyl thiourées, on obtient les produits de formule (I) réunis dans le tableau 5.

TABLEAU 5

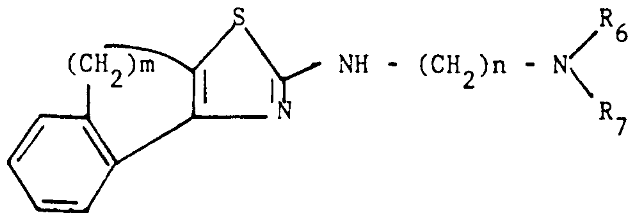

| Ex N° | N° Code SR | m | n | 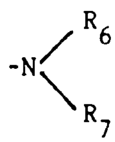 | Sel F°C (solvant) |
|---|---|---|---|---|---|
| 119 | 44317 A | 3 | 2 | 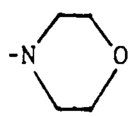 | Dichlorhydrate 217 (iPrOH-EtOH 95) |
| 120 | 44323 A | 3 | 2 | 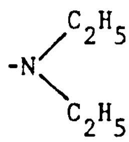 | Dichlorhydrate 170 (iPrOH-Et2O) |
| 121 | 44326 A | 3 | 3 | " | Dichlorhydrate 0,5 $H_2O$ 192 (iPrOH-$iPr_2O$) |
| 122 | 44327 A | 3 | 3 | 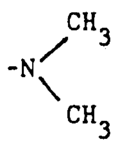 | Dichlorhydrate 1 $H_2O$ 198 ($Et_2O$) |
| 123 | 44392 A | 3 | 3 | 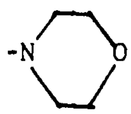 | Dichlorhydrate 0,5 $H_2O$ 245 dec ($Et_2O$) |
| 124 | 44393 A | 2 | 2 | 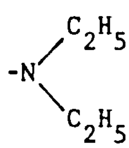 | Dichlorhydrate 0,5 $H_2O$ 181 ($Et_2O$-PrOH) |

EXEMPLE 125 :

[(Diéthylamino-2 éthyl) amino]-2 nitro-9 dihydro-5,6 4H benzo [6,7] cyclohepta [1,2-d] thiazole, dichlorhydrate (SR 44 411 A).

On opère comme dans l'exemple 118 à partir de N-benzoyl N'-(diéthylamino-3 propyl) thiourée d'une part et de bromo-2 nitro-8 benzocycloheptanone-1 d'autre part.

De la même façon on obtient le produit attendu sous forme de dichlorhydrate.

F : 192-195 °C (isopropanol-éthanol à 95).

Les produits selon l'invention ont été étudiés en ce qui concerne leurs propriétés pharmacologiques et en particulier en ce qui concerne leur affinité pour les récepteurs cholinergiques muscariniques.

L'étude a été effectuée par des tests biochimiques in vitro et également par des tests pharmacologiques réalisés chez l'animal.

ETUDE BIOCHIMIQUE IN VITRO

Chez les mammifères, il existe deux sous classes de récepteurs cholinergiques muscariniques : les récepteurs $M_1$ et $M_2$

Les réceptuers de type $M_1$ sont concentrés dans certaines zones de cerveau telles que l'hippocampe, le cortex cérébral, le striatum ainsi que dans les ganglions sympatiques. Ces sites de liaison peuvent être sélectivement marqués par la pirenzépine tritiée ($^3$H-PZ). Ceux de type $M_2$ prédominent dans le coeur et dans l'iléon et peuvent être marqués par la N-méthylscopolamine tritiée ($^3$H-NMS). Afin de déterminer la sélectivité des produits de l'invention, vis-à-vis des sites $M_1$ et $M_2$, on a étudié leur intéraction in vitro avec les fixations à haute affinité de la $^3$H-PZ et de la $^3$H-NMS sur des membranes d'hippocampe de rat et de muscle lisse d'iléon de cobaye, respectivement.

Methodologies

A) Recherche d'une affinité pour le récepteur cholinergique muscarinique de type $M_1$.

L'intéraction des molécules avec les récepteurs muscariniques de type $M_1$ a été étudiée en mesurant in vitro sur un homogénat d'hippocampe de rat, le déplacement de la pirenzépine tritiée de ses sites de fixation spécifiques. Des aliquots (10 µl) d'un homogénat d'hippocampe de rat à 5 % (P/v) dans un tampon $Na_2HPO_4$ (50 mM, pH 7,40), sont incubés 2 heures à 4 °C en présence de$^3$ H-PZ (76 Ci/mmole ; 1 nM final) et de concentrations croissantes en produit à étudier. Le volume final est de 2 ml. La réaction est arrêtée par centrifugation 10 minutes à 50.000 xg. Après décantation et lavage des culots, la radioactivité fixée est comptée par scintillation liquide. La fixation non spécifique est déterminée en présence de 10 µM de sulfate d'atropine. La concentration inhibitrice 50 ($CI_{50}$) est déterminée graphiquement.

(Réf. : WatsonJ.D., Roeskoe W.R. and Yamamura H.I., Life Sci., 1982, 31, 2019-2029).

B) Recherche d'une affinité pour le récepteur cholinergique muscarinique de type $M_2$ .

L'intéraction avec les récepteurs muscariniques de type $M_2$ a été étudiée en mesurant in vitro sur homogénat de muscle lisse d'iléon de cobaye, le déplacement de la N-méthyl-scopolamine tritiée de ses sites de fixation spécifiques. Des aliquotes (50 µl) d'un homogénat de muscle lisse d'iléon de cobaye à 0,625 % P/v) dans 20 mM de tampon HEPES : acide ((hydroxy-2 éthyl-4) pipérazine-1-yl)-2 éthanesulfonique, contenant NaCl (100 mM) et Mg $Cl_2$ (10 mM) (pH final : 7,5), sont incubés 20 minutes à 30 °C en présence de $^3$H-NMS (85 Ci/mmole ; 0,3 nM final) et de concentrations croissantes en produits à tester. Le volume final est de 1 ml. La réaction est arrêtée par centrifugation 5 minutes à 15.000 xg. La fixation non spécifique est déterminée en présence de 10 mM de sulfate d'atropine.

(Réf. : Hammer R. et al., Nature, 1980, 283, 90-92 ; Hulme E.C. et al., Mol. Pharmacol., 1978, 14, 737-750).

Resultats

Le tableau 6 indique les affinités des produits de l'invention pour les récepteurs $M_1$ et $M_2$ . Les résultats sont exprimés en concentrations inhibitrices 50 pour cent ($CI_{50}$), soit la concentration (en µM) qui induit un déplacement de 50 % du ligand tritié fixé aux récepteurs membranaires. La $CI_{50}$ de déplacement de la $^3$H-PZ représente l'affinité pour le récepteur $M_1$ ; la $CI_{50}$ de déplacement de la $^3$H-NMS représente l'affinité pour le récepteur $M_2$.

De plus dans la colonne suivante de tableau on a indiqué le rapport R entre les $CI_{50}$ pour les récepteurs $M_1$ et $M_2$ qui exprime la sélectivité des produits vis-à-vis de l'un des types de récepteurs.

A titre de comparaison, on a indiqué dans le tableau 6 les résultats obtenus avec 3 produits de référence.

TABLEAU 6

| N° de Produit | $^3$H-PZ($M_1$) $CI_{50}$ µM | $^3$H-NMS($M_2$) $CI_{50}$ µM | R = $CI_{50}(M_2)$ / $CI_{50}(M_1)$ |
|---|---|---|---|
| SR 44284 A | 8 | 40 | 5 |
| SR 44286 A | 6 | 100 | 16 |
| SR 44318 A | 3,4 | 38 | 11 |
| SR 44323 A | 1,7 | 8,5 | 5 |
| SR 44326 A | 2,2 | > 100 | > 45 |
| SR 44327 A | 1,9 | 38 | 20 |
| SR 44345 A | 1 | 70 | 70 |
| SR 44346 A | 2 | 25 | 12,5 |
| SR 44347 A | 0,35 | 13 | 37 |
| SR 44372 A | 0,12 | 5 | 41 |
| SR 44373 A | 0,70 | 15 | 21 |
| SR 44374 A | 0,40 | 8 | 20 |
| SR 44393 A | 2,7 | 42 | 15 |
| SR 44411 A | 0,9 | 6,7 | 7 |
| SR 44421 A | 3,6 | > 100 | > 27 |
| SR 44422 A | 8,1 | 100 | 12 |
| SR 44423 A | 6,4 | 100 | 15 |
| SR 44434 A | 1,5 | 70 | 46 |
| SR 44435 A | 0,36 | 3 | 8 |
| SR 44436 A | 1,7 | 50 | 29 |
| SR 44437 A | 0,43 | 3 | 7 |
| SR 44438 A | 1 | 60 | 60 |
| SR 44451 A | 6 | 80 | 13 |
| SR 44465 A | 2,6 | 40 | 15 |
| SR 44467 A | 1,5 | 38 | 25 |

| | | | |
|---|---|---|---|
| SR 44468 A | 1,2 | 22 | 18 |
| SR 44469 A | 2,6 | 60 | 23 |
| SR 44488 A | 1,7 | 34 | 20 |
| SR 44489 A | 0,3 | 2 | 6 |
| SR 44490 A | 1,5 | 25 | 16 |
| SR 44491 A | 0,8 | 8,5 | 10 |
| SR 44492 A | 3,6 | 28 | 8 |
| SR 44493 A | 1,6 | 45 | 28 |
| SR 44494 A | 0,8 | 80 | 100 |
| SR 44514 A | 0,5 | > 100 | > 200 |
| SR 44515 A | 0,2 | 3,4 | 17 |
| SR 44516 A | 3,4 | 75 | 22 |
| SR 44573 A | 1,8 | 13 | 7 |
| SR 44574 A | 0,2 | 24 | 120 |
| SR 44575 A | 7 | > 100 | > 14 |
| SR 44749 A | 1,8 | 26 | 14 |
| SR 44769 A | 2 | 30 | 15 |
| SR 44770 A | 0,6 | 26 | 43 |
| SR 44786 A | 0,6 | 40 | 67 |
| SR 44791 A | 5 | 40 | 8 |
| SR 44792 A | 1,8 | 30 | 17 |
| SR 44813 A | 6 | > 100 | > 17 |
| SR 44814 A | 1,4 | 10 | 7 |
| SR 44832 A | 2,4 | > 100 | > 42 |
| SR 44839 A | 0,4 | 100 | 250 |
| SR 44840 A | 0,6 | > 100 | > 167 |
| SR 44856 A | 0,3 | > 100 | > 300 |
| SR 44862 A | 3 | 34 | 11 |
| SR 44864 A | 0,28 | > 100 | > 360 |
| SR 44886 A | 6,3 | 70 | 11 |
| SR 44904 A | 6 | > 100 | > 17 |
| SR 44905 A | 2,4 | 60 | 25 |
| SR 44906 A | 2,4 | > 100 | > 42 |

| | | | |
|---|---|---|---|
| SR 44908 A | 0,13 | 4 | 31 |
| SR 44915 A | 0,4 | 40 | 100 |
| SR 44916 A | 0,13 | 5 | 38 |
| SR 44963 A | 0,12 | 6 | 50 |
| SR 44964 A | 0,9 | 28 | 31 |
| SR 44965 A | 1,2 | 60 | 50 |
| SR 44966 A | 4,5 | 100 | 22 |
| SR 44967 A | 1,8 | 40 | 22 |
| SR 45001 A | 2,60 | 30 | 11 |
| SR 45015 A | 0,3 | 4 | 13 |
| SR 45016 A | 0,10 | 5,5 | 55 |
| SR 45030 A | 1,60 | 80 | 50 |
| SR 45106 A | 3 | > 100 | > 33 |
| Oxotrémorine | 0,24 | 0,2 | 1 |
| Arécoline | 17 | 4,5 | 0,3 |
| Pilocarpine | 2,5 | 7 | 3 |

Les résultats montrent que les composés selon l'invention présentent une forte affinité pour les récepteurs cholinergiques muscariniques avec une spécificité marquée pour les récepteurs centraux de type $M_1$.

ETUDE PHARMACOLOGIQUE IN VIVO

La pirenzépine (PZ) est un antagoniste spécifique des récepteurs cholinergiques muscariniques centraux $M_1$. L'injection intrastriatale de PZ chez la souris induit un comportement rotatoire. On a étudié l'antagonisme de ce comportement par les produits selon l'invention.

Les produits selon l'invention sont administrés soit par voie intrapéritonéale (i.p.) soit par voie orale (p.o.) après avoir été solubilisés dans l'eau distillée ou mis en suspension dans une solution de gomme arabique à 5 %. Les contrôles sont réalisés par administration du solvant pur dans les mêmes conditions.

Les animaux utilisés sont des souris femelles (Swiss, CD 1, Charles River, France) de poids corporel compris entre 25 et 30 grammes.

La pirenzépine est mise en solution dans un tampon phosphate, le pH de la solution est 6.

Les produits à étudier ou leur solvants sont administrés soit par injection par voie i.p., soit par voie orale par une sonde oesophagienne, sous un volume de 0,4 ml pour 20 g de poids corporel. L'administration a lieu soit 15 minutes (voie i.p.) soit 30 minutes (p.o.) avant une injection directe de pirenzépine à la dose de 1µg dans 1µl de solvant dans le striatum droit de la souris, selon la méthode décrite par P. WORMS et al. dans Eur. J. Pharmacol., 1986, 121, 395-401.

Le nombre de rotations contralatérales (sens opposé au cotê de l'injection) a été compté pendant trois périodes de 2 minutes après injection de pirenzépine : minutes 2 à 4, 8 à 10 et 13 à 15. Chaque traitement comporte 3 à 4 doses et 10 animaux par dose. Pour chaque traitement, on calcule le nombre total de rotations et le pourcentage d'antagonisme vis-à-vis du lot contrôle.

Pour chaque produit on détermine graphiquement la dose efficace 50 ($DE_{50}$) : dose qui diminue de 50 % le nombre de rotations induites par la pirenzépine. Les résultats sont reportés dans le tableau 7.

Le tableau 7 indique pour chaque produit testé, la dose efficace 50 ($DE_{50}$) en mg/kg chez la souris pour l'antagonisme des rotations induites par la pirenzépine soit par voie i.p. soit par voie orale.

A titre de comparaison, on a reporté les résultats obtenus avec 3 produits de référence.

TABLEAU 7

| Produits n° | Antagonisme pirenzépine $DE_{50}$ mg/kg i.p. | Antagonisme pirenzépine $DE_{50}$ mg/kg p.o. |
|---|---|---|
| SR 44244 A | 8 | 20 |
| SR 44284 A | 8 | - |
| SR 44285 A | 3 | - |
| SR 44286 A | 3 | 10 |
| SR 44318 A | 1,5 | 3 |
| SR 44323 A | 10 | - |
| SR 44326 A | 3 | - |
| SR 44327 A | 10 | - |
| SR 44345 A | 0,5 | 1 |
| SR 44346 A | 1 | - |
| SR 44347 A | 0,03 | 0,20 |
| SR 44372 A | 1 | 3 |
| SR 44373 A | 0,3 | 1 |
| SR 44374 A | 3 | - |
| SR 44392 A | 3 | - |
| SR 44393 A | 3 | - |
| SR 44411 A | 15 | - |
| SR 44421 A | - | 0,2 |
| SR 44435 A | 2 | - |
| SR 44467 A | - | 3 |
| SR 44493 A | - | 0,3 |
| SR 44494 A | - | 2 |
| SR 44514 A | 3 | - |
| SR 44573 A | - | 2,50 |
| SR 44749 A | - | 3 |

| | | |
|---|---|---|
| SR 44770 A | - | 3 |
| SR 44792 A | - | 3 |
| SR 44814 A | - | 0,2 |
| SR 44839 A | - | 0,2 |
| SR 44856 A | - | 3 |
| SR 44862 A | - | 3 |
| SR 44887 A | - | 0,3 |
| SR 44915 A | - | 3 |
| SR 44949 A | - | 10 |
| SR 44964 A | - | 3 |
| SR 44965 A | - | 3 |
| SR 45001 A | - | 3 |
| SR 45015 A | - | 0,70 |
| SR 45016 A | - | 2 |
| SR 45030 A | - | 1 |
| Oxotrémorine * | 0,005 | - |
| Arécoline * | 1 | 0,5 |
| Pilocarpine * | 1 | 10 |

* Forte induction d'effets secondaires (tremblements, salivation, lacrymation, défécation, piloérection, hypothermie, sédation) à doses proches des doses actives dans ces tests.

Les résultats du tableau 7 montrent que les composés selon l'invention possèdent une activité stimulante de la transmission cholinergique centrale et sont donc susceptibles d'être utilisés comme agonistes des récepteurs muscariniques.

Par ailleurs certains des produits selon l'invention ont révélé une activité antagoniste de l'effet des acides aminés excitateurs dans le cerveau. Cette activité a été mesurée dans le test de libération de l'acétylcholine provoquée par l'acide N-méthyl-D-aspartique (NMDA) sur des coupes de striatum de rat (Lehmann J. et Scatton B. Brain Research 1982, 252, 77-89).

Enfin la toxicité aigue a été déterminée pour divers produits selon l'invention. Les produits ont été administrés par voie i.p. à doses croissantes à des lots de 10 souris femelles (Swiss, CD 1, Charles River, France) de poids corporel 20 g

1. La mortalité provoquée par les produits étudiés a été notée pendant les 24 heures suivant l'administration du produit. On a déterminé pour chacun des produits la dose léthale 50 ($DL_{50}$), c'est à dire la dose provoquant la mort de 50 % des animaux.

Les résultats obtenus sont réunis dans le tableau 8 ci-dessous.

TABLEAU 8

| Produits n° | $DL_{50}$ (mg/kg) i.p. |
|---|---|
| SR 44244 A | 250 |
| SR 44273 A | > 100 |
| SR 44284 A | 450 |
| SR 44286 A | 200 |
| SR 44345 A | 200 |
| SR 44347 A | 60 |
| SR 44372 A | 75 |
| SR 44373 A | 75 |
| SR 44374 A | 150 |

Les produits selon l'invention sont donc peu toxiques et ne manifestent aucun signe de toxicité aux doses où ils sont actifs.

Par suite les composés (I) peuvent être utilisés en tant que médicaments.

Les résultats indiqués ci-dessus permettent d'envisager l'utilisation des produits selon l'invention pour le traitement des syndromes dégénératifs liés à la sénescence et notamment les troubles de la mémoire et les démences séniles.

Selon un autre de ses aspects la présente demande concerne donc les compositions pharmaceutiques contenant au moins un des composés de formule (I) ou un de leurs sels en tant qu'ingrédient actif.

Dans les compositions pharmaceutiques de la présente invention pour l'administration orale, sublinguale, transdermique ou rectale, les ingrédients actifs de formule I ci-dessus peuvent être administrés sous forme unitaires d'administration, en mélange avec les supports pharmaceutiques classiques, aux êtres humains notamment pour le traitement des démences séniles. Les formes unitaires d'administration appropriées comprennent les formes par voie orale, telles que les comprimés, les gélules les poudres, les granulés et les solutions ou suspensions orales, les formes d'administration sublinguale et buccale, les formes d'administration sous-cutanée, intramusculaire ou intraveineuse et les formes d'administration rectale.

Afin d'obtenir l'effet désiré, la dose de principe actif peut varier entre 20 et 500 mg par jour.

Chaque dose unitaire peut contenir de 5 à 200 mg d'ingrédient actif en combinaison avec un support pharmaceutique. Cette dose unitaire peut être administrée 1 à 4 fois par jour.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les poudres ou les granulés dispersibles dans l'eau peuvent contenir l'ingrédient actif en mélange avec des agents de dispersion ou des agents mouillants, ou des agents de mise en suspension comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectable qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

A titre d'exemple de préparation galénique on peut préparer des gélules contenant :

| | |
|---|---|
| SR 44421 A | 0,010 g |
| Lactose | 0,050 g |
| Stéarate de magnésium | 0,005 g |

en mélangeant intimement les ingrédients ci-dessus et en versant le mélange dans des gélules de gélatine dure.

**Revendications**

**Revendications pour les Etats contractants suivants : AT, BE, CH, DE, FR, GB, IT, LI, LU, NL, SE**

**1.** Nouveaux dérivés du thiazole de formule générale :

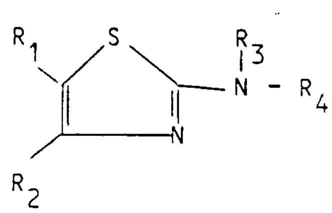

(I)

dans laquelle :

- $R_1$ et $R_2$ représentent chacun indépendamment l'hydrogène ; un groupe alkyle en $C_1$-$C_4$ ; un groupe phényle ; un groupe phényle substitué une ou plusieurs fois par un atome d'halogène, ou par un groupe alkyle en $C_1$-$C_4$, ou par un groupe alcoxy en $C_1$-$C_4$, un groupe nitro, un groupe hydroxy ; ou l'un des groupes $R_1$ et $R_2$ désigne l'hydrogène et l'autre représente : un groupe naphtyle ; un groupe benzyle ; un groupe $\alpha,\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

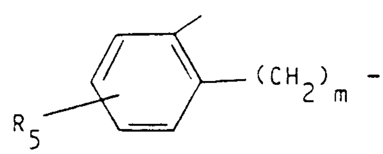

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ;
avec la condition que si l'un des groupes $R_1$ ou $R_2$ désigne l'hydrogène l'autre est différent de H ou méthyle ;
- $R_3$ représente l'hydrogène ou un groupe alkyle en $C_1$-$C_4$
- $R_4$ représente :
- un groupe

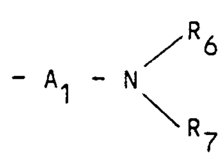

dans lequel $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine ;

- un groupe

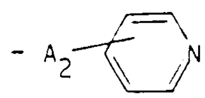

ou $A_2$ désigne un groupe $(CH_2)_m$ avec m = 0, 1, 2, 3 qui substitue le cycle pyridine en position 2, 3 ou 4 ;

- un groupe

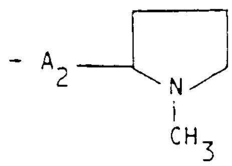

où $A_2$ est comme indique ci-dessus

- un groupe

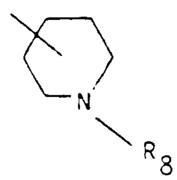

où $R_8$ désigne un groupe alkyle en $C_1$-$C_4$ ou encore le substituant

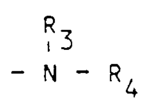

représente un groupe

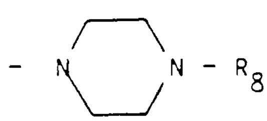

où $R_8$ est comme indiqué ci-dessus à la condition dans ce cas, que si $R_1$ représente l'hydrogène ou un groupe alkyle en $C_1$-$C_4$, $R_2$ est autre qu'un groupe alkyle en $C_1$-$C_4$ ou qu'un groupe cyclohexyle, ainsi que leurs sels d'addition avec les acides minéraux ou organiques pharmaceutiquement acceptables.

**2.** Composés selon la revendication 1, caractérisés en ce que $R_1$ et $R_2$ représentent chacun indépendamment un groupe phényle substitué une ou plusieurs fois par un atome de chlore, de fluor ou un groupe méthyle.

3. Composés selon l'une des revendications 1 ou 2, caractérisés en ce que $R_4$ représente le groupe

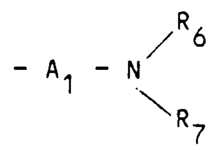

dans lequel
$A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés avec l' atome d' azote auquel ils sont liés constituent un hétérocycle à 5 ou 6 chaînons contenant éventuellement un second hétéroatome et notamment les cycles pyrrolidine, pipéridine, morpholine et N-alkylpiperazine.

4. Composés selon l'une quelconque des revendications 1 à 3, caractérisés en ce que $R_4$ représente un groupe

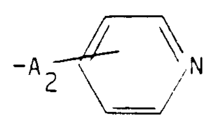

dans lequel
$A_2$ désigne un groupe $(CH_2)_m$ avec m = 0, 1, 2, 3 qui substitue le cycle pyridine en position 2, 3 ou 4.

5. Composés selon l'une quelconque des revendications 1 à 3, caractérisés en ce que $R_4$ représente un groupe

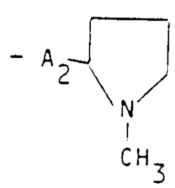

où $A_2$ est tel que défini ci-dessus.

6. Composés selon l'une quelconque des revendications 1 à 5, caractérisés en ce que $R_1$ et $R_2$ désignent chacun indépendamment l'hydrogène ; un groupe alkyle en $C_1$-$C_4$ ; un groupe phényle ; un groupe phényle substitué une ou plusieurs fois par un atome d'halogène, un groupe alkyle en $C_1$-$C_4$, un groupe nitro ou un groupe hydroxy ou l'un des groupes $R_1$ et $R_2$ désigne l'hydrogène et l'autre représente un groupe naphtyle ; un groupe benzyle ; un groupe $\alpha,\alpha$-diméthyl-benzyle, un groupe cyclohexyle, un groupe biphényle ou un groupe adamantyle avec la condition que si l'un des groupes $R_1$ ou $R_2$ désigne l'hydrogène l'autre est différent de H ou méthyle.

7. Composés selon l'une quelconque des revendications 1 à 5, caractérisés en ce que $R_1$ et $R_2$ considérés ensemble représentent un groupe

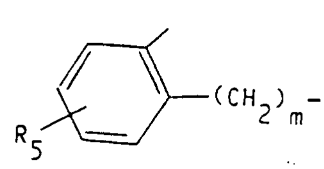

où le groupe phényle substitue le thiazole en position 4 et le groupe $(CH_2)_m$ - en position 5 et dans

lequel m représente un entier égal à 2 ou 3 et $R_5$ désigne l'hydrogène ou un groupe nitro.

**8.** Dérivé du thiazole de formule (I) :

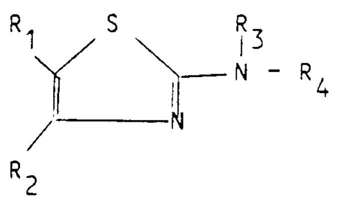

(I)

dans laquelle :

$R_1$ est le groupe phényle ;
$R_2$ est le groupe benzyle ;
$R_3$ est l'hydrogène et
$R_4$ est le groupe $(CH_2)_3$ - $N(C_2H_5)_2$ sous la forme du dichlorhydrate.

**9.** Procédé de préparation des composés de formule I, caractérisé en ce qu'on chauffe en milieu acide de pH 1 à 6 une thiourée substituée de formule :

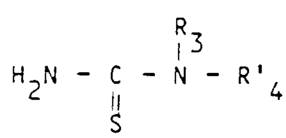

où $R_3$ a la signification indiquée ci-dessus et $R'_4$ a les mêmes significations que $R_4$ sauf dans les cas où $R_4$ contient une amine primaire ou secondaire où $R'_4$ désigne alors le groupe correspondant à $R_4$ dans lequel un hydrogène de ladite fonction amine a été remplacé par un groupe protecteur résistant à l'hydrolyse en milieu alcalin, avec un dérivé carbonylé alphabromé de formule :

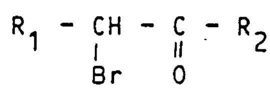

ou avec le dérivé bromé correspondant dans lequel le groupe carbonyle est protégé sous forme d'acétal, pour obtenir les composés (I) et qu'éventuellement on salifie le composé I selon un procédé connu.

**10.** Compositions pharmaceutiques contenant à titre de principe actif un composé de formule (I) ou l'un de ses sels pharmaceutiquement acceptables selon l'une des revendications 1 à 8, en combinaison avec un véhicule pharmaceutiquement acceptable.

**11.** Compositions pharmaceutiques selon la revendication 10, contenant de 20 à 500 mg de principe actif.

**12.** Utilisation des dérivés du thiazole de formule :

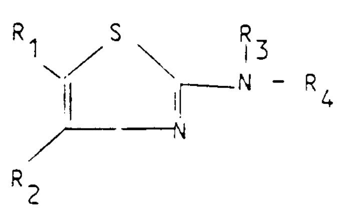

(I)

dans laquelle :

- $R_1$ et $R_2$ représentent chacun indépendamment l'hydrogène ; un groupe alkyle en $C_1$-$C_4$ ; un groupe phényle ; un groupe phényle substitué une ou plusieurs fois par un atome d'halogène, ou par un groupe alkyle en $C_1$-$C_4$, ou par un groupe alcoxy en $C_1$-$C_4$, un groupe nitro, un groupe hydroxy ; ou l'un des groupes $R_1$ et $R_2$ désigne l'hydrogène et l'autre représente : un groupe naphtyle ; un groupe benzyle ; un groupe $\alpha$,$\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

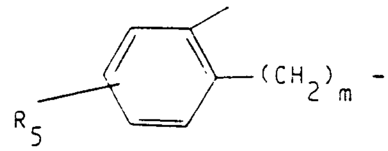

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ; avec la condition que si t'un des groupes $R_1$ ou $R_2$ désigne l'hydrogène l'autre est différent de H ou méthyle ;
- $R_3$ représente l'hydrogène ou un groupe alkyle en $C_1$-$C_4$
- $R_4$ représente :
- un groupe

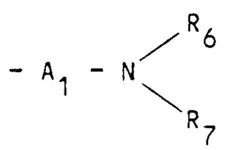

dans lequel $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine₁ morpholine et N-alkylpipérazine ;
- un groupe

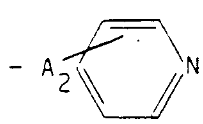

ou $A_2$ désigne un groupe $(CH_2)_m$ avec m = 0, 1, 2, 3 qui substitue le cycle pyridine en position 2, 3 ou 4 ;
- un groupe

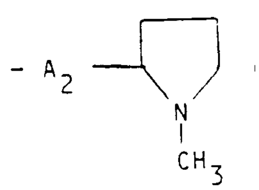

où $A_2$ est comme indiqué ci-dessus

- un groupe

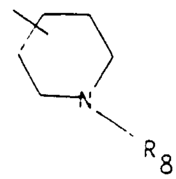

ou $R_8$ désigne un groupe alkyle en $C_1-C_4$ ou encore le substituant

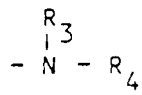

représente un groupe

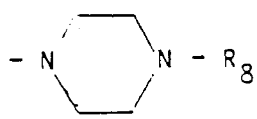

où $R_8$ est comme indiqué ci-dessus ; ou de leurs sels d'addition avec les acides minéraux ou organiques pharmaceutiquement acceptables pour la préparation de médicaments utiles pour le traitement des syndromes dégénératifs liés à la sénescence.

**Revendications pour l'Etat contractant suivant : ES**

**1.** Procédé pour l'obtention de dérivés du thiazole actifs sur le système cholinergique répondant à la formule générale :

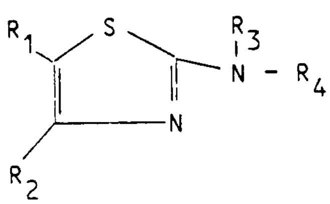

(I)

dans laquelle :

- $R_1$ et $R_2$ représentent chacun indépendamment l'hydrogène ; un groupe alkyle en $C_1$-$C_4$ ; un groupe phényle ; un groupe phényle substitué une ou plusieurs fois par un atome d'halogène, ou par un groupe alkyle en $C_1$-$C_4$, ou par un groupe alcoxy en $C_1$-$C_4$, nitro, hydroxy ; ou l'un des groupes $R_1$ et $R_2$ désigne l'hydrogène et l'autre représente : un groupe naphtyle ; un groupe benzyle ; un groupe $\alpha,\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

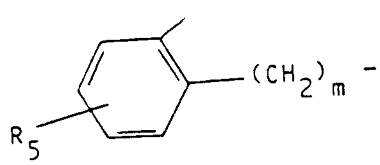

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ;
avec la condition que si l'un des groupes $R_1$ ou $R_2$ désigne l'hydrogène l'autre est différent de H ou méthyle :

- $R_3$ représente l'hydrogène ou un groupe alkyle en $C_1$-$C_4$
- $R_4$ représente :
- un groupe

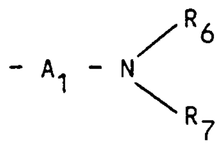

dans lequel $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine ;

- un groupe

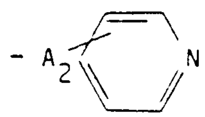

ou $A_2$ désigne un groupe $(CH_2)_m$ avec m = 0, 1, 2, 3 qui substitue le cycle pyridine en position 2, 3 ou 4 ;

- un groupe

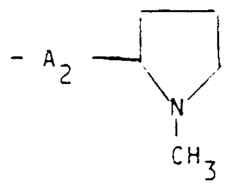

où $A_2$ est comme indiqué ci-dessus

- un groupe

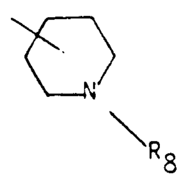

où $R_8$ désigne un groupe alkyle en $C_1$-$C_4$ ou encore le substituant

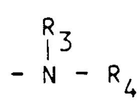

représente un groupe

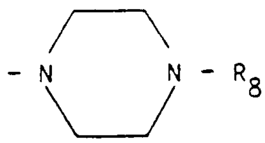

où $R_8$ est comme indiqué ci-dessus à la condition dans ce cas, que si $R_1$ représente l'hydrogène ou un groupe alkyle en $C_1-C_4$, $R_2$ est autre qu'un groupe alkyle en $C_1-C_4$ ou qu'un groupe cyclohexyle,

ainsi que leurs sels d'addition avec les acides minéraux ou organiques pharmaceutiquement acceptables, caractérisé en ce qu'on chauffe en milieu acide de pH 1 à 6 une thiourée substituée de formule :

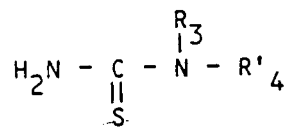

où $R_3$ a la signification indiquée ci-dessus et $R'_4$ a les mêmes significations que $R_4$ sauf dans les cas où $R_4$ contient une amine primaire ou secondaire où $R'_4$ désigne alors le groupe correspondant à $R_4$ dans lequel un hydrogène de ladite fonction amine a été remplacé par un groupe protecteur résistant à l'hydrolyse en milieu alcalin.

avec un dérivé carbonylé alphabromé de formule :

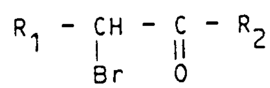

ou avec le dérivé bromé correspondant dans lequel le groupe carbonyle est protégé sous forme d'acétal, pour obtenir les composés (I) et

qu'éventuellement on salifie le compose I selon un procédé connu.

**2.** Procédé selon la revendication 1, caractérisé en ce que l'on utilise un dérivé carbonylé, alphabromé, dans lequel $R_1$ et $R_2$ représentent chacun indépendamment un groupe phényle substitué une ou plusieurs fois par un atome de chlore, de fluor ou un groupe méthyle.

**3.** Procédé selon l'une des revendications 1 ou 2, caractérisé en ce que $R'_4$ représente le groupe :

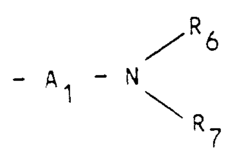

dans lequel

$A_1$ désigne un groupe alkyle droit ou ramifié en $C_2$-$C_5$ $R_6$ et $R_7$ considérés indépendamment représentent un groupe protecteur, l'hydrogène, un groupe alkyle en $C_1-C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine.

**4.** Procédé selon l'une des revendications 1 ou 2, caractérisé en ce que R'$_4$ représente un groupe :

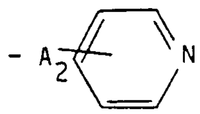

dans lequel

A$_2$ désigne un groupe $(CH_2)_m$ avec m = 0, 1, 2, 3 qui substitue le cycle pyridine en position 2, 3 ou 4.

**5.** Procédé selon l'une des revendications 1 ou 2, caractérisé en ce que R'$_4$ représente un groupe

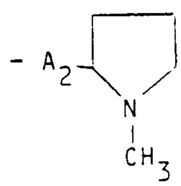

où A$_2$ est tel que défini dans la revendication 1.

**6.** Procédé selon l'une quelconque des revendications 1 à 5, caractérisé en ce que R$_1$ et R$_2$ désignent chacun indépendamment l'hydrogène ; un groupe alkyle en $C_1$-$C_4$ ; un groupe phényle ; un groupe phényle substitué une ou plusieurs fois par un atome d'halogène, un groupe alkyle en $C_1$-$C_4$, un groupe nitro ou un groupe hydroxy ou l'un des groupes R$_1$ et R$_2$ désigne l'hydrogène et l'autre représente un groupe naphtyle ; un groupe benzyle ; un groupe $\alpha,\alpha$-diméthyl-benzyle, un groupe cyclohexyle, un groupe biphényle ou un groupe adamantyle avec la condition que si l'un des groupes R$_1$ ou R$_2$ désigne l'hydrogène l'autre est différent de H ou méthyle.

**7.** Procédé selon l'une quelconque des revendications 1 à 5, caractérisé en ce que R$_1$ et R$_2$ considérés ensemble représentent un groupe

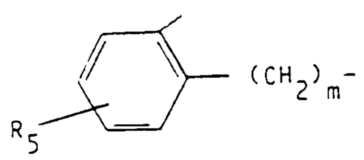

où le groupe phényle substitue le thiazole en position 4 et le groupe $(CH_2)_m$ - en position 5 et dans lequel m représente un entier égal à 2 ou 3 et R$_5$ désigne l'hydrogène ou un groupe nitro.

**8.** Procédé pour l'obtention du dérivé du thiazole de formule (I) :

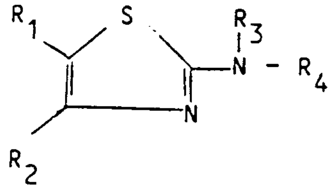

(I)

dans laquelle :

R$_1$ est le groupe phényle ;

R$_2$ est le groupe benzyle ;

$R_3$ est l'hydrogène et

$R_4$ est le groupe $(CH_2)_3$ - $N(C_2H_5)_2$

sous la forme du dichlorhydrate, caractérisé en ce qu'il consiste à chauffer en milieu acide de pH 1 à 6 une thiourée substituée de formule :

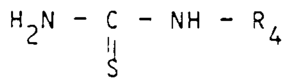

dans laquelle $R_4$ est le groupe $-(CH_2)_3-N(C_2H_5)_2$ avec le dérivé carbonylé alphabromé de formule :

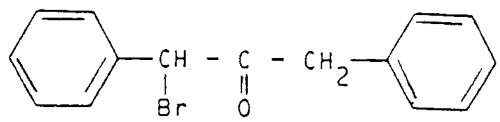

et à transformer le composé ainsi obtenu en le dichlorhydrate.

**9.** Utilisation des dérivés du thiazole de formule :

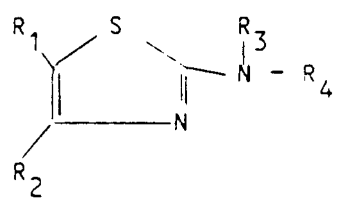

(I)

dans laquelle :

- $R_1$ et $R_2$ représentent chacun indépendamment l'hydrogène ; un groupe alkyle en $C_1-C_4$ ; un groupe phényle ; un groupe phényle substitué une ou plusieurs fois par un atome d'halogène, ou par un groupe alkyle en $C_1-C_4$, ou par un groupe alcoxy en $C_1-C_4$, un groupe nitro, un groupe hydroxy ; ou l'un des groupes $R_1$ et $R_2$ désigne l'hydrogène et l'autre représente : un groupe naphtyle ; un groupe benzyle ; un groupe $\alpha,\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

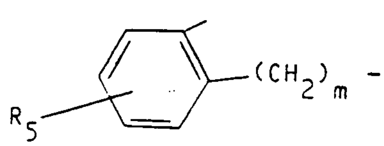

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ;

avec la condition que si l'un des groupes $R_1$ ou $R_2$ désigne l'hydrogène l'autre est différent de H ou méthyle ;
- $R_3$ représente l'hydrogène ou un groupe alkyle en $C_1$-$C_4$
- $R_4$ représente :

- un groupe

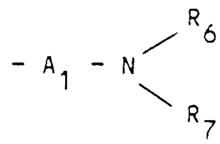

dans lequel $A_1$ désigne un groupe alkyle droit ou ramifié en $C_2$-$C_5$ $R_6$ et $R_7$ considérés indépendamment représentent l'hydrogène, un groupe alkyle en $C_1-C_4$, un groupe cycloalkyle en $C_3$-$C_6$ ou encore $R_6$ et $R_7$ considérés avec l'atome d'azote auquel ils sont liés constituent un hétérocyle à 5 ou 6 chaînons contenant éventuellement un second hétéroatome et notamment les cycles pyrrolidine, pipéridine, morpholine et N-alkylpipérazine ;
- un groupe

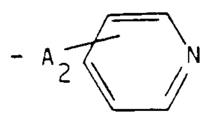

ou $A_2$ désigne un groupe $(CH_2)_m$ avec m = 0, 1, 2, 3 qui substitue le cycle pyridine en position 2, 3 ou 4 ;
- un groupe

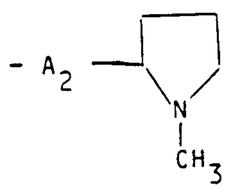

où $A_2$ est comme indiqué ci-dessus
- un groupe

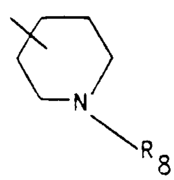

où $R_8$ désigne un groupe alkyle en $C_1-C_4$ ou encore le substituant

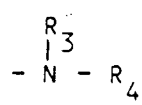

représente un groupe

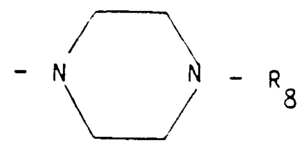

où $R_8$ est comme indiqué ci-dessus ; ou de l'un de leurs sels d'addition avec les acides minéraux ou orqaniques pharmaceutiquement acceptables pour la préparation de médicaments utiles pour le traitement des syndromes dégénératifs liés à la sénescence.

**Revendications pour l'Etat contractant suivant : GR**

**1.** Nouveaux dérivés du thiazole de formule générale :

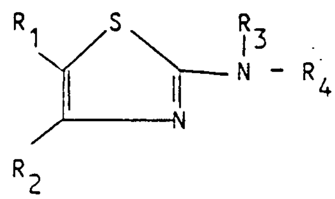

(I)

dans laquelle :

- $R_1$ et $R_2$ représentent chacun indépendamment l'hydrogéne ; un groupe alkyle en $C_1$-$C_4$ ; un groupe phényle ; un groupe phényle substitué une ou plusieurs fois par un atome d'halogène, ou par un groupe alkyle en $C_1$-$C_4$, ou par un groupe alcoxy en $C_1$-$C_4$, un groupe nitro, un groupe hydroxy ; ou l'un des groupes $R_1$ et $R_2$ désigne l'hydrogène et l'autre représente : un groupe naphtyle ; un groupe benzyle ; un groupe $\alpha,\alpha$-diméthyl-benzyle ; un groupe cyclohexyle ; un groupe biphényle ; un groupe thiényle ; un groupe adamantyle, ou encore $R_1$ et $R_2$ considérés ensemble représentent un groupe :

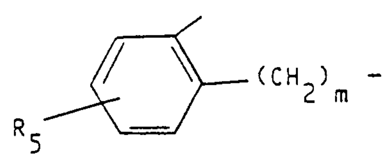

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ;
avec la condition que si l'un des groupes $R_1$ ou $R_2$ désigne l'hydrogène l'autre est différent de H ou méthyle ;
- $R_3$ représente l'hydrogène ou un groupe alkyle en $C_1$-$C_4$
- $R_4$ représente :
- un groupe

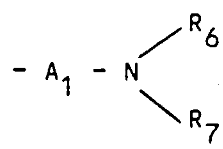

dans lequel $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés `avec l'atome d'azote auquel ils sont liés constituent un hétérocycle à 5 ou 6 chaînons contenant éventuellement un second hétéroatome et notamment

les cycles pyrrolidine, pipéridine, morpholine et N-alkylpipérazine ;

- un groupe

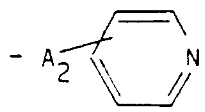

ou $A_2$ désigne un groupe $(CH_2)_m$ avec m = 0, 1, 2, 3 qui substitue le cycle pyridine en position 2, 3 ou 4 ;

- un groupe

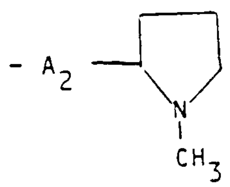

où $A_2$ est comme indiqué ci-dessus

- un groupe

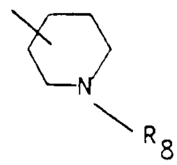

où $R_8$ désigne un groupe alkyle en $C_1$-$C_4$ ou encore le substituant

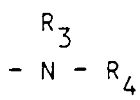

représente un groupe

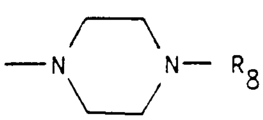

où $R_8$ est comme indiqué ci-dessus à la condition dans ce cas, que si $R_1$ représente l'hydrogène ou un groupe alkyle en $C_1$-$C_4$, $R_2$ est autre qu'un groupe alkyle en $C_1$-$C_4$ ou qu'un groupe cyclohexyle, ainsi que leurs sels d'addition avec les acides minéraux ou organiques pharmaceutiquement acceptables.

**2.** Composés selon la revendication 1, caractérisés en ce que $R_1$ et $R_2$ représentent chacun indépendamment un groupe phényle substitué une ou plusieurs fois par un atome de chlore, de fluor ou un groupe méthyle.

**3.** Composés selon l'une des revendications 1 ou 2, caractérisés en ce que $R_4$ représente le groupe

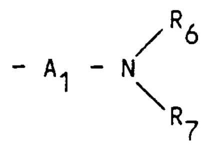

dans lequel

$A_1$ désigne un groupe alkyle droit ou ramifié en $C_2$-$C_5$ $R_6$ et $R_7$ considérés indépendamment représentent l'hydrogène, un groupe alkyle en $C_1-C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine.

**4.** Composés selon l'une quelconque des revendications 1 à 3, caractérisés en ce que $R_4$ représente un groupe

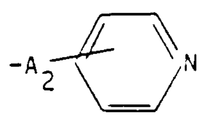

dans lequel

$A_2$ désigne un groupe $(CH_2)_m$ avec m = 0, 1, 2, 3 qui substitue le cycle pyridine en position 2, 3 ou 4.

**5.** Composés selon l'une quelconque des revendications 1 à 3, caractérisés en ce que $R_4$ représente un groupe

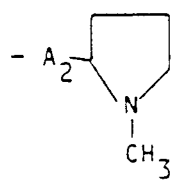

où $A_2$ est tel que défini ci-dessus.

**6.** Composés selon l'une quelconque des revendications 1 à 5, caractérisés en ce que $R_1$ et $R_2$ désignent chacun indépendamment l'hydrogène ; un groupe alkyle en $C_1-C_4$ ; un groupe phényle ; un groupe phényle substitué une ou plusieurs fois par un atome d'halogène, un groupe alkyle en $C_1-C_4$, un groupe nitro ou un groupe hydroxy ou l'un des groupes $R_1$ et $R_2$ désigne l'hydrogène et l'autre représente un groupe naphtyle ; un groupe benzyle ; un groupe $\alpha,\alpha$-diméthyl-benzyle, un groupe cyclohexyle, un groupe biphényle ou un groupe adamantyle avec la condition que si l'un des groupes $R_1$ ou $R_2$ désigne l'hydrogène l'autre est différent de H ou méthyle.

**7.** Composés selon l'une quelconque des revendications 1 à 5, caractérisés en ce que $R_1$ et $R_2$ considérés ensemble représentent un groupe

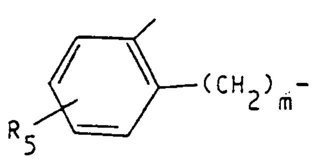

où le groupe phényle substitue le thiazole en position 4 et le groupe $(CH_2)_m$ - en position 5 et dans lequel m représente un entier égal à 2 ou 3 et $R_5$ désigne l'hydrogène ou un groupe nitro.

**8.** Dérivé du thiazole de formule (I) :

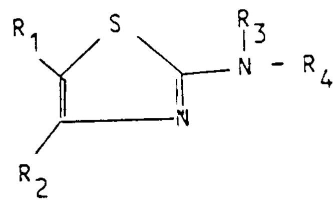

(I)

dans laquelle :

$R_1$ est le groupe phényle ;
$R_2$ est le groupe benzyle ;
$R_3$ est l'hydrogène et
$R_4$ est le groupe $(CH_2)_3$ - $N(C_2H_5)_2$ sous la forme du dichlorhydrate.

**9.** Procédé de préparation des composés de formule I, caractérisé en ce qu'on chauffe en milieu acide de pH 1 à 6 une thiourée substituée de formule :

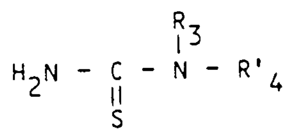

où $R_3$ a la signification indiquée ci-dessus et $R'_4$ a les mêmes significations que $R_4$ sauf dans les cas où $R_4$ contient une amine primaire ou secondaire où $R'_4$ désigne alors le groupe correspondant à $R_4$ dans lequel un hydrogène de ladite fonction amine a été remplacé par un groupe protecteur résistant à l'hydrolyse en milieu alcalin, avec un dérivé carbonylé alphabromé de formule :

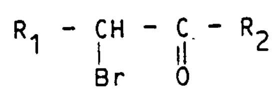

ou avec le dérivé bromé correspondant dans lequel le groupe carbonyle est protégé sous forme d'acétal, pour obtenir les composés (I) et qu'éventuellement on salifie le composé I selon un procédé connu.

**10.** Utilisation des dérivés du thiazole de formule :

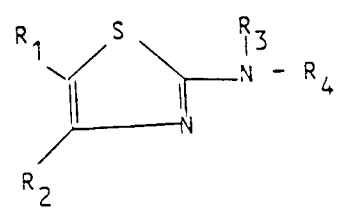

(I)

dans laquelle :

- $R_1$ et $R_2$ représentent chacun indépendamment l'hydrogène ; un groupe alkyle en $C_1$-$C_4$ ; un groupe pnényle ; un groupe phényle substitué une ou plusieurs fois par un atome d'halogène, ou par un groupe alkyle en $C_1$-$C_4$, ou par un groupe alcoxy en $C_1$-$C_4$, un groupe nitro, un groupe

hydroxy ; ou l'un des groupes $R_1$ et $R_2$ désigne l'hydrogène et l'autre représente : un groupe naphtyle ; un groupe benzyle ; un groupe $\alpha,\alpha$ = diméthyl-benzyle ; un groupe cyclohexyle ; un groupe biphényle ; un groupe thiényle ; un groupe adamantyle, ou encore $R_1$ et $R_2$ considérés ensemble représentent un groupe :

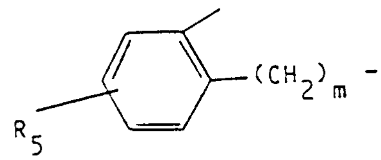

où le groupe phényle est lié en position 4 du thiazole et le groupe $(CH_2)_m$ en position 5 et dans lequel m représente un nombre entier égal à 2 ou 3 et $R_5$ désigne l'hydrogène ou un groupe nitro occupant l'une des positions libres du cycle ;
avec la condition que si l'un des groupes $R_1$ ou $R_2$ désigne l'hydrogène l'autre est différent de H ou méthyle ;

- $R_3$ représente l'hydrogène ou un groupe alkyle en $C_1$-$C_4$
- $R_4$ représente :
- un groupe

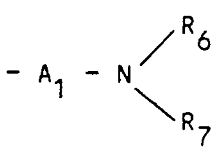

dans lequel $A_1$ désigne un groupe alkyle droit ou ramifié en $C_2$-$C_5$ $R_6$ et $R_7$ considérés indépendamment représentent l'hydrogène, un groupe alkyle en $C_1$-$C_4$, un groupe cycloalkyle en $C_3$-$C_6$ ou encore $R_6$ et $R_7$ considérés avec l'atome d'azote auquel ils sont liés constituent un hétérocycle à 5 ou 6 chaînons contenant éventuellement un second hétéroatome et notamment les cycles pyrrolidine, pipéridine, morpholine et N-alkylpipérazine ;

- un groupe

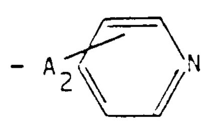

ou $A_2$ désigne un groupe $(CH_2)_m$ avec m = 0, 1, 2, 3 qui substitue le cycle pyridine en position 2, 3 ou 4 ;

- un groupe

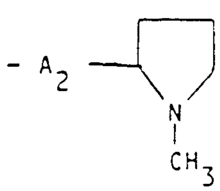

où $A_2$ est comme indiqué ci-dessus

- un groupe

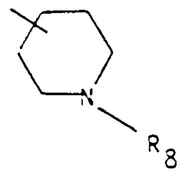

où $R_8$ désigne un groupe alkyle en $C_1$-$C_4$ ou encore le substituant

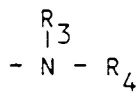

représente un groupe

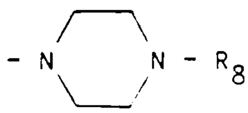

où $R_8$ est comme indiqué ci-dessus ; ou de l'un de leurs sels d'addition avec des acides minéraux ou organiques pharmaceutiquement acceptables pour la préparation de médicaments utiles pour le traitement des syndromes dégénératifs liés à la sénescence.

**Claims**

**Claims for the following Contracting States : AT, BE, CH, DE, FR, GB, IT, LI, LU, NL, SE**

**1.** Novel thiazole derivatives of the general formula:

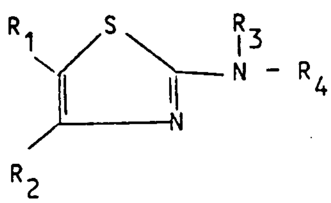

(I)

in which:

- $R_1$ and $R_2$ each independently represent hydrogen; a $C_1$-$C_4$ alkyl group; a phenyl group; a phenyl group monosubstituted or polysubstituted by a halogen atom, or by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha, alpha-dimethylbenzyl group; a cyclohexyl group; a biphenyl group; a thienyl group; an adamantyl group; or alternatively $R_1$ and $R_2$, taken together, represent a group:

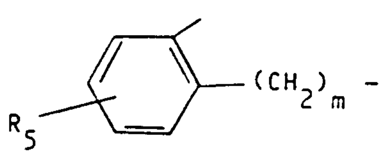

in which the phenyl group is bonded to the 4-position of the thiazole and the group

$$-(CH_2)_m-$$

to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;

- $R_3$ represents hydrogen or a $C_1-C_4$ alkyl group;
- $R_4$ represents:
- a group:

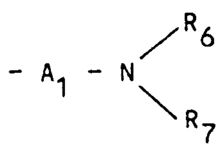

in which $A_1$ denotes a linear or branched $C_2$-$C_5$ alkyl group; and $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings;

- a group:

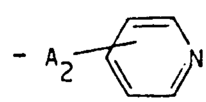

in which $A_2$ denotes a group $(CH_2)_m$, where m = 0, 1, 2 or 3, which substitutes the pyridine ring in the 2-, 3- or 4-position;

- a group:

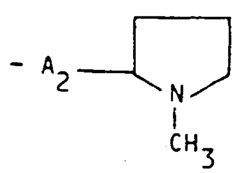

in which $A_2$ is as indicated above; or

- a group:

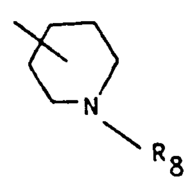

in which $R_8$ denotes a $C_1$-$C_4$ alkyl group; or alternatively the substituent

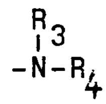

represents a group:

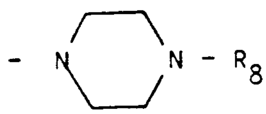

in which $R_8$ is as indicated above, with the proviso that if $R_1$ represents hydrogen or a $C_1$-$C_4$ alkyl group, then $R_2$ is other than a $C_1$-$C_4$ alkyl group or a cyclohexyl group, and also their pharmaceutically acceptable addition salts with mineral or organic acids.

**2.** Compounds according to claim 1, characterized in that $R_1$ and $R_2$ each independently represent a phenyl group monosubstited or polysubstituted by a chlorine atom, a fluorine atom or a methyl group.

**3.** Compounds according to one of claims 1 or 2, characterized in that $R_4$ represents a group:

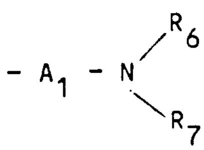

in which $A_1$ denotes a linear or branched $C_2$-$C_5$ alkyl group; $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings.

**4.** Compounds according to any one of claims 1 to 3, characterized in that $R_4$ represents a group:

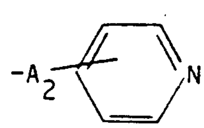

in which $A_2$ denotes a group $(CH_2)_m$, where m = 0, 1, 2 or 3, which substitutes the pyridine ring in the 2-, 3- or 4-position.

**5.** Compounds according to anyone of claims 1 to 3, characterized in that $R_4$ represents a group:

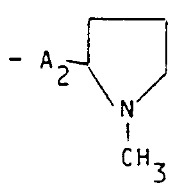

in which $A_2$ is as defined above.

6. Compounds according to any one of claims 1 to 5, characterized in that $R_1$ and $R_2$ each independently denote hydrogen, a $C_1$-$C_4$ alkyl group; a phenyl group; a phenyl group monosubstituted or polysubstituted by a halogen atom; a $C_1$-$C_4$ alkyl group, a nitro group or a hydroxyl group; or one of the groups $R_1$-$R_2$ denotes hydrogen and the other represents a naphthyl group; a benzyl group; an alpha, alpha-dimethylbenzyl group; a cyclohexyl group; a biphenyl group or an adamantyl group, with the proviso that if one of the groups $R_1$ or $R_2$ denotes hydrogen, the other is different from H or methyl.

7. Compounds according to anyone of claims 1 to 5, characterized in that $R_1$ and $R_2$, taken together, represent a group:

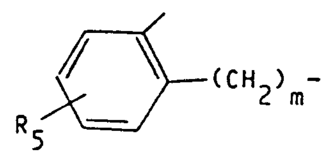

in which the phenyl group substitutes the thiazole in the 4-position and the group $(CH_2)_m$ substitutes the thiazole in the 5-position, and in which m represents an integer equal to 2 or 3 and $R_5$ denotes hydrogen or a nitro group.

8. Thiazole derivative of formula (I):

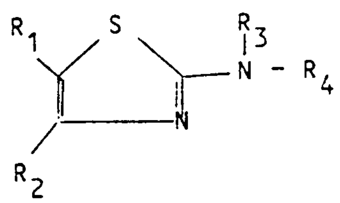

(I)

in which:
- $R_1$ is the phenyl group;
- $R_2$ is the benzyl group;
- $R_3$ is hydrogen and
- $R_4$ is the group $(CH_2)_3$-$N(C_2H_5)_2$ under the form of the dihydrochloride.

9. Process for the preparation of the compounds of formula I, characterized in that a substituted thiourea of the formula:

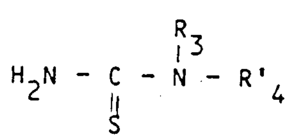

in which $R_3$ has the meaning indicated above and $R'_4$ has the same meanings as $R_4$ except in cases where $R_4$ contains a primary or secondary amine, when $R'_4$ denotes the group corresponding to $R_4$ in which a hydrogen belonging to the said amine group has been replaced by a protective group which is resistant to hydrolysis in alkaline medium, is heated, in an acid medium of pH 1 to 6, with an alpha-brominated carbonyl derivative of the formula:

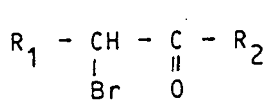

or with the corresponding bromine derivative in which the carbonyl group is protected in the form of an

acetal, to give the compounds (I), and wherein the compound I is optionally converted to a salt by a known process.

**10.** Pharmaceutical compositions containing, as the active principle, a compound of the formula (I) or one of its pharmaceutically acceptable salts according to any one of claims 1 to 8, in combination with a pharmaceutically acceptable vehicle.

**11.** Pharmaceutical compositions according to claim 10, containing from 20 to 500 mg of active principle.

**12.** Use of the thiazole derivatives of formula:

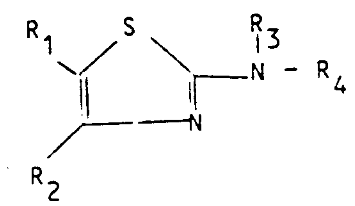

(I)

in which:

- $R_1$ and $R_2$ each independently represent hydrogen; a $C_1$-$C_4$ alkyl group; a phenyl group; a phenyl group monosubstituted or polysubstituted by a halogen atom, or by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha, alpha-dimethylbenzyl group; a cyclohexyl group; a biphenyl group; a thienyl group; an adamantyl group; or alternatively $R_1$ and $R_2$, taken together, represent a group:

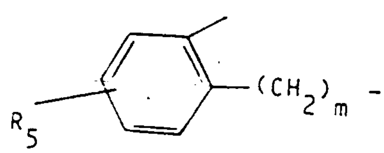

in which the phenyl group is bonded to the 4-position of the thiazole and the group

$$-(CH_2)_m-$$

to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;
- $R_3$ represents hydrogen or a $C_1$-$C_4$ alkyl group;
- $R_4$ represents:
- a group:

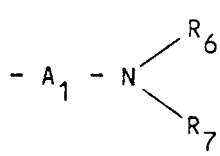

in which $A_1$ denotes a linear or branched $C_2$-$C_5$ alkyl group; $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine,

morpholine and N-alkyl-piperazine rings;

- a group:

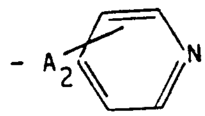

in which $A_2$ denotes a group $(CH_2)_m$, where m = 0, 1, 2 or 3, which substitutes the pyridine ring in the 2-, 3- or 4-position;

- a group:

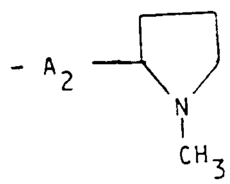

in which $A_2$ is as indicated above; or

- a group:

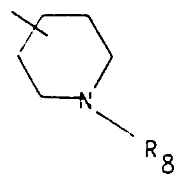

in which $R_8$ denotes a $C_1$-$C_4$ alkyl group; or alternatively the substituent

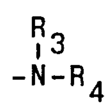

represents a group:

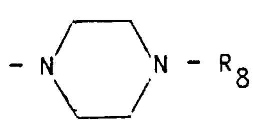

in which $R_8$ is as indicated above or of one of their pharmaceutically acceptable addition salts with mineral or organic acids, for the preparation of drugs useful for the treatment of degenerative syndromes associated with senescence.

**Claims for the following Contracting State : ES**

**1.** Process for the obtention of thiazole derivatives active on the cholinergic system, having the general formula:

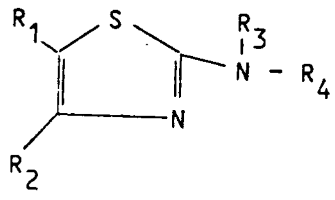

(I)

in which:

- $R_1$ and $R_2$ each independently represent hydrogen; a $C_1$-$C_4$ alkyl group; a phenyl group; a phenyl group monosubstituted or polysubstituted by a halogen atom, or by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha,alpha-dimethylbenzyl group; a cyclohexyl group; a biphenyl group; a thienyl group; an adamantyl group; or alternatively $R_1$ and $R_2$, taken together, represent a group:

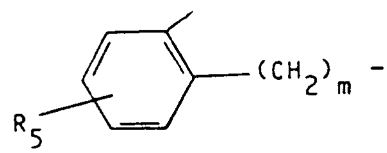

in which the phenyl group is bonded to the 4-position of the thiazole and the group

$$-(CH_2)_m-$$

to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;
- $R_3$ represents hydrogen or a $C_1$-$C_4$ alkyl group;
- $R_4$ represents:
- a group:

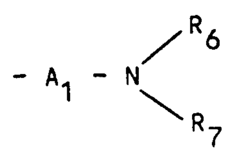

in which $A_1$ denotes a linear or branched $C_2$-$C_5$ alkyl group; and $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkylpiperazine rings;
- a group:

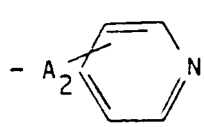

in which $A_2$ denotes a group $(CH_2)_m$, where m = 0, 1, 2 or 3, which substitutes the pyridine ring in the 2-, 3- or 4-position;

- a group:

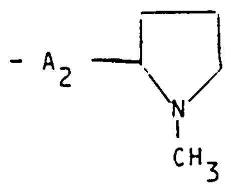

in which $A_2$ is as indicated above; or

- a group:

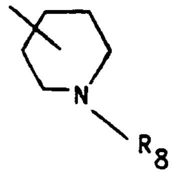

in which $R_8$ denotes a $C_1$-$C_4$ alkyl group; or alternatively the substituent

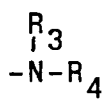

represents a group:

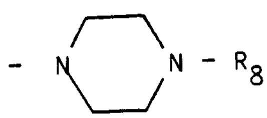

in which $R_8$ is as indicated above, with the proviso that if $R_1$ represents hydrogen or a $C_1$-$C_4$ alkyl group, then $R_2$ is other than a $C_1$-$C_4$ alkyl group or a cyclohexyl group,
and also their pharmaceutically acceptable addition salts with mineral or organic acids, characterized in that a substituted thiourea of the formula:

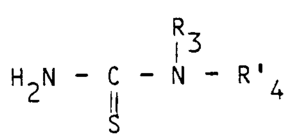

in which $R_3$ has the meaning indicated above and $R'_4$ has the same meanings as $R_4$ except in cases where $R_4$ contains a primary or secondary amine, when $R'_4$ denotes the group corresponding to $R_4$ in which a hydrogen belonging to the said amine group has been replaced by a protective group which is resistant to hydrolysis in alkaline medium, is heated, in an acid medium of pH 1 to 6, with an alpha-brominated carbonyl derivative of the formula:

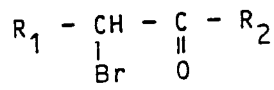

or with the corresponding bromine derivative in which the carbonyl group is protected in the form of an acetal, to give the compounds (I), and wherein the compound I is optionally converted to a salt by a known process.

**2.** Process according to claim 1, characterized in that an alpha-brominated carbonyl derivative, in which $R_1$ and $R_2$, each independently represent a phenyl group monosubstituted or polysubstituted by a chlorine atom, a fluorine atom or a methyl group, is used.

**3.** Process according to one of claims 1 or 2, characterized in that $R'_4$ represents a group:

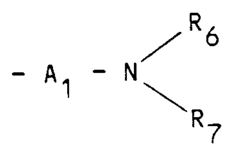

in which $A_1$ denotes a linear or branched $C_2$-$C_5$ alkyl group; and $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkylpiperazine rings.

**4.** Process according to one of claims 1 or 2, characterized in that $R'_4$ represents a group:

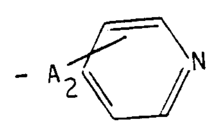

in which $A_2$ denotes a group $(CH_2)_m$, where m = 0, 1, 2 or 3, which substitutes the pyridine ring in the 2-, 3- or 4-position.

**5.** Process according to one of claim 1 or 2, characterized in that $R'_4$ represents a group:

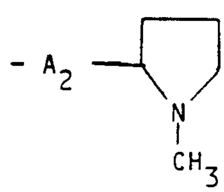

in which $A_2$ is as defined in claim 1.

**6.** Process according to any one of claims 1 to 5, characterized in that $R_1$ and $R_2$ each independently denote hydrogen; a $C_1$-$C_4$ alkyl group; a phenyl group; a phenyl group monosubstituted or polysubstituted by a halogen atom, a $C_1$-$C_4$ alkyl group, a nitro group or a hydroxyl group; or one of the groups $R_1$ and $R_2$ denotes hydrogen and the other represents a naphthyl group; a benzyl group; an alpha,alpha-dimethylbenzyl group; a cyclohexyl group; a biphenyl group or an adamantyl group, with the proviso that if one of the groups $R_1$ and $R_2$ denotes hydrogen, the other is different from H or methyl.

**7.** Process according to any one of claims 1 to 5, characterized in that $R_1$ and $R_2$, taken together, represent a group:

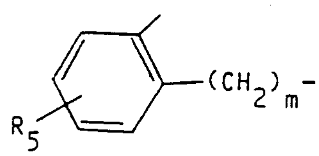

in which the phenyl group substitutes the thiazole in the 4-position and the group

$$-(CH_2)_m-$$

substitutes the thiazole in the 5-position, and in which m represents an integer equal to 2 or 3 and $R_5$ denotes hydrogen or a nitro group.

**8.** Process for the obtention of a thiazole derivative of formula (I):

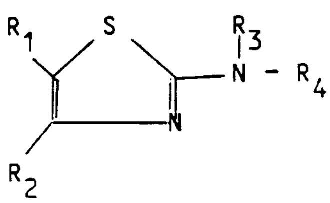

(I)

in which:

- $R_1$ is the phenyl group;
- $R_2$ is the benzyl group;
- $R_3$ is hydrogen and
- $R_4$ is the group $(CH_2)_3$-$N(C_2H_5)_2$ in the form off the dihydrochloride, characterized in that a substituted thiourea of formula:

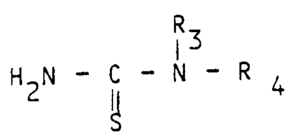

in which $R_4$ is the group $-(CH_2)_3$-$N(C_2H_5)_2$ is reacted with an alpha-brominated carbonyl derivative of formula:

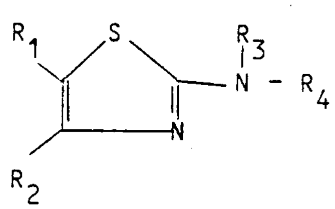

(I)

and the thus obtained compound is converted into the dihyrochloride.

**9.** Use of the thiazole derivatives of formula: in which:

- $R_1$ and $R_2$ each independently represent hydrogen; a $C_1$-$C_4$ alkyl group; a phenyl group; a phenyl group monosubstituted or polysubstituted by a halogen atom, or by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha,alpha-dimethylbenzyl group; a cyclohexyl group; a biphenyl group; a thienyl group; an adamantyl group; or alternatively $R_1$ and $R_2$, taken together, represent a group:

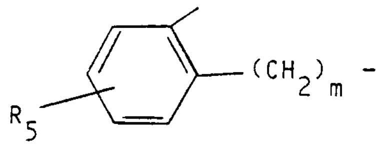

in which the phenyl group is bonded to the 4-position of the thiazole and the group $(CH_2)_m$ to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;
- $R_3$ represents hydrogen or a $C_1$-$C_4$ alkyl group;
- $R_4$ represents:
- a group:

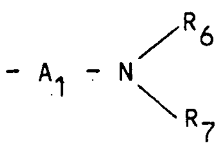

in which $A_1$ denotes a linear or branched $C_2$-$C_5$ alkyl group and $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings;
- a group:

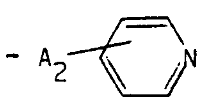

in which $A_2$ denotes a group $(CH_2)_m$, where m = 0, 1, 2 or 3, which substitutes the pyridine ring in the 2-, 3- or 4-position;
- a group:

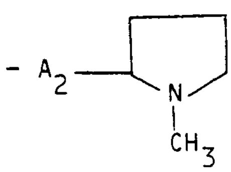

in which $A_2$ is as indicated above; or

- a group:

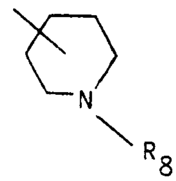

in which $R_8$ denotes a $C_1$-$C_4$ alkyl group; or alternatively the substituent

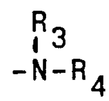

represents a group:

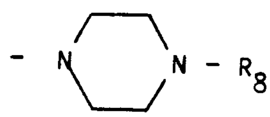

in which $R_8$ is as indicated above, or of one of their pharmaceutically acceptable addition salts with mineral or organic acids for the preparation of drugs useful for the treatment of degenerative syndromes associated with senescence.

**Claims for the following Contracting State : GR**

**1.** Novel thiazole derivatives of the general formula:

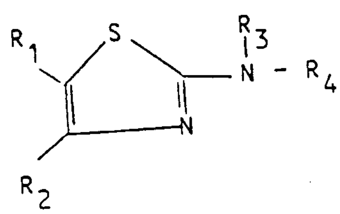

(I)

in which:

- $R_1$ and $R_2$ each independently represent hydrogen; a $C_1$-$C_4$ alkyl group; a phenyl group; a phenyl group monosubstituted or polysubstituted by a halogen atom, by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha, alpha-dimethylbenzyl group; a cyclohexyl group; a biphenyl group; a thienyl group; an adamantyl group; or alternatively $R_1$ and $R_2$, taken together, represent a group:

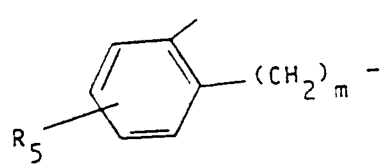

in which the phenyl group is bonded to the 4-position of the thiazole and the group $-(CH_2)_m-$ to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;

- $R_3$ represents hydrogen or a $C_1$-$C_4$ alkyl group;
- $R_4$ represents:
- a group:

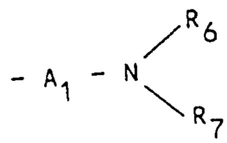

in which $A_1$ denotes a linear or branched $C_2$-$C_5$ alkyl group; and $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings;

- a group:

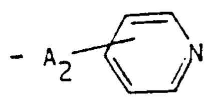

in which $A_2$ denotes a group $(CH_2)_m$, where m = 0, 1, 2 or 3, which substitutes the pyridine ring in the 2-, 3- or 4-position;

- a group:

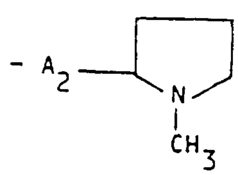

in which $A_2$ is as indicated above; or

- a group:

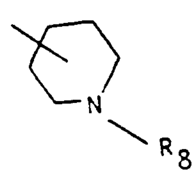

in which $R_8$ denotes a $C_1$-$C_4$ alkyl group; or alternatively the substituent

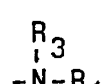

represents a group:

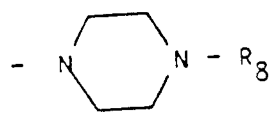

in which $R_8$ is as indicated above, with the proviso that if $R_1$ represents hydrogen or a $C_1$-$C_4$ alkyl group, then $R_2$ is other than a $C_1$-$C_4$ alkyl group or a cyclohexyl group, and also their pharmaceutically acceptable addition salts with mineral or organic acids.

**2.** Compounds according to claim 1, characterized in that $R_1$ and $R_2$ each independently represent a phenyl group monosubstited or polysubstituted by a chlorine atom, a fluorine atom or a methyl group.

**3.** Compounds according to one of claims 1 or 2, characterized in that $R_4$ represents a group:

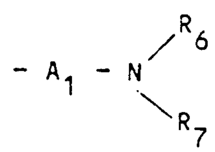

in which $A_1$ denotes a linear or branched $C_2$-$C_5$ alkyl group; $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings.

**4.** Compounds according to any one of claims 1 to 3, characterized in that $R_4$ represents a group:

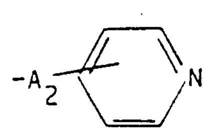

in which $A_2$ denotes a group $(CH_2)_m$, where m = 0, 1, 2 or 3, which substitutes the pyridine ring in the 2-, 3- or 4-position.

**5.** Compounds according to any one of claims 1 to 3, characterized in that $R_4$ represents a group:

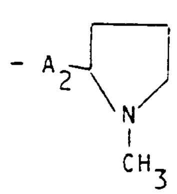

in which $A_2$ is as defined above.

**6.** Compounds according to any one of claims 1 to 5, characterized in that $R_1$ and $R_2$ each independently denote hydrogen; a $C_1$-$C_4$ alkyl group; a phenyl group; a phenyl group monosubstituted or polysubstituted by a halogen atom; a $C_1$-$C_4$ alkyl group, a nitro group or a hydroxyl group; or one of the groups $R_1$-$R_2$ denotes hydrogen and the other represents a naphthyl group; a benzyl group; an alpha, alpha-dimethylbenzyl group; a cyclohexyl group; a biphenyl group or an adamantyl group, with the proviso

that if one of the groups $R_1$ or $R_2$ denotes hydrogen, the other is different from H or methyl.

**7.** Compounds according to any one of claims 1 to 5, characterized in that $R_1$ and $R_2$, taken together, represent a group:

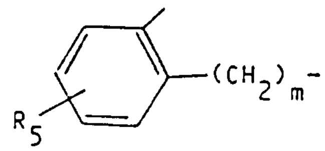

in which the phenyl group substitutes the thiazole in the 4-position and the group $(CH_2)_m$ substitutes the thiazole in the 5-position, and in which m represents an integer equal to 2 or 3 and $R_5$ denotes hydrogen or a nitro group.

**8.** Thiazole derivative of formula (I):

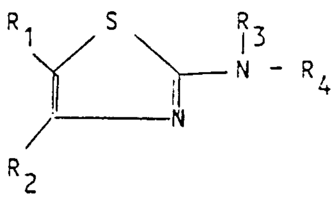

(I)

in which:
- $R_1$ is the phenyl group;
- $R_2$ is the benzyl group;
- $R_3$ is hydrogen and
- $R_4$ is the group $(CH_2)_3$-$N(C_2H_5)_2$ under the form of the dihydrochloride.

**9.** Process for the preparation of the compounds of formula I, characterized in that a substituted thiourea of the formula:

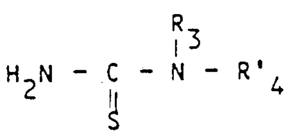

in which $R_3$ has the meaning indicated above and $R'_4$ has the same meanings as $R_4$ except in cases where $R_4$ contains a primary or secondary amine, when $R'_4$ denotes the group corresponding to $R_4$ in which a hydrogen belonging to the said amine group has been replaced by a protective group which is resistant to hydrolysis in alkaline medium, is heated, in an acid medium of pH 1 to 6, with an alpha-brominated carbonyl derivative of the formula:

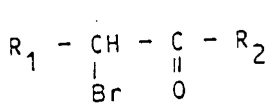

or with the corresponding bromine derivative in which the carbonyl group is protected in the form of an acetal, to give the compounds (I) , and wherein the compound I is optionally converted to a salt by a known process.

**10.** Use of the thiazole derivatives of formula:

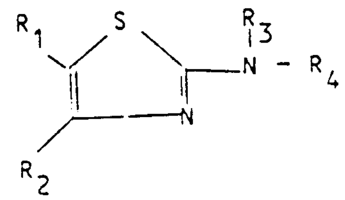

(I)

in which:

- $R_1$ and $R_2$ each independently represent hydrogen; a $C_1$-$C_4$ alkyl group; a phenyl group; a phenyl group monosubstituted or polysubstituted by a halogen atom, by a $C_1$-$C_4$ alkyl group, or by a $C_1$-$C_4$ alkoxy group, a nitro group or an hydroxyl group; or one of the groups $R_1$ and $R_2$ denotes hydrogen and the other represents a naphthyl group; a benzyl group; an alpha, alpha-dimethylbenzyl group; a cyclohexyl group; a biphenyl group; a thienyl group; an adamantyl group; or alternatively $R_1$ and $R_2$, taken together, represent a group:

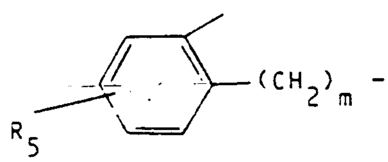

in which the phenyl group is bonded to the 4-position of the thiazole and the group

$$-(CH_2)_m-$$

to the 5-position, and in which m represents an integer equal to 2 or 3 and $R_5$ denotes hydrogen or a nitro group occupying one of the free positions on the ring, with the proviso that if one of the groups $R_1$ or $R_2$ denotes hydrogen, the other is different from H or methyl;
- $R_3$ represents hydrogen or a $C_1$-$C_4$ alkyl group;
- $R_4$ represents:
- a group:

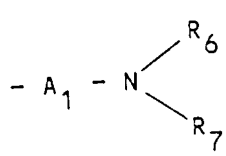

in which $A_1$ denotes a linear or branched $C_2$-$C_5$ alkyl group; $R_6$ and $R_7$, taken independently, represent hydrogen, a $C_1$-$C_4$ alkyl group or a $C_3$-$C_6$ cycloalkyl group, or alternatively $R_6$ and $R_7$, taken with the nitrogen atom to which they are bonded, form a 5-membered or 6-membered heterocycle optionally containing a second heteroatom, and especially the pyrrolidine, piperidine, morpholine and N-alkyl-piperazine rings;
- a group:

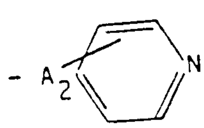

in which $A_2$ denotes a group $(CH_2)_m$, where m = 0, 1, 2 or 3, which substitutes the pyridine ring

in the 2-, 3- or 4-position;

- a group:

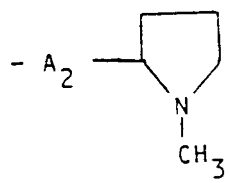

in which $A_2$ is as indicated above; or

- a group:

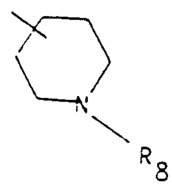

in which $R_8$ denotes a $C_1$-$C_4$ alkyl group; or alternatively the substituent

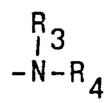

represents a group:

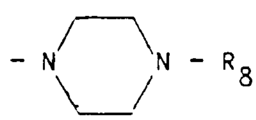

in which $R_8$ is as indicated above or of one of their pharmaceutically acceptable addition salts with mineral or organic acids, for the preparation of drugs useful for the treatment of degenerative syndromes associated with senescence.

**Patentansprüche**

**Patentansprüche für folgenge Vertragsstaaten : AT, BE, CH, DE, FR, GB, IT, LI, LU, NL, SE**

**1.** Neue Thiazolderivate der allgemeinen Formel

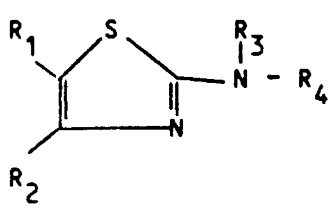

(I)

worin:

- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe

darstellt; oder eine der Gruppen $R_1$ und $R_2$ Wasserstoff bezeichnet und die andere eine Naphthyl-; Benzyl-; α,α-Dimethylbenzyl-; Cyclohexyl-; Diphenyl-; Thienyl- oder Adamantylgruppe darstellt; oder aber $R_1$ und $R_2$ zusammen eine Gruppe

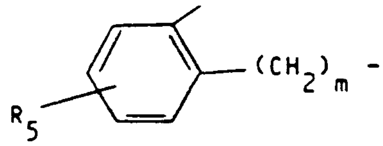

darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet; mit der Maßgabe, daß, wenn eine der Gruppen $R_1$ oder $R_2$ Wasserstoff ist, die andere nicht H oder Methyl ist;

- $R_3$ Wasserstoff oder eine $C_1$-$C_4$-Alkylgruppe darstellt;
- $R_4$ darstellt:
  - eine Gruppe

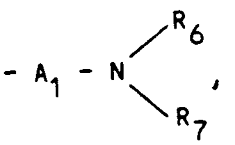

worin $A_1$ eine gerade oder verzweigte $C_2$-$C_5$-Alkylgruppe bezeichnet,
$R_6$ und $R_7$ unabhängig voneinander Wasserstoff, eine $C_1$–$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein zweites Heteroatom enthält, insbesondere die Pyrrolidin-, Piperidin-, Morpholin- und N-Alkylpiperazinringe;

  - eine Gruppe

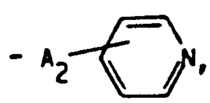

worin $A_2$ eine Gruppe $(CH_2)_m$ mit m = 0, 1, 2, 3 ist, die den Pyridinring in Stellung 2, 3 oder 4 substituiert;

  - eine Gruppe

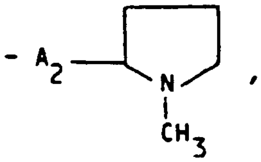

worin $A_2$ wie oben definiert ist;

- eine Gruppe

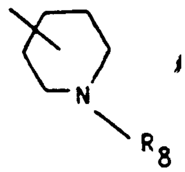

worin $R_8$ eine $C_1$-$C_4$-Alkylgruppe ist; oder aber der Substituent

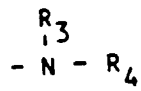

eine Gruppe

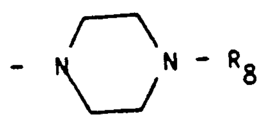

darstellt, worin $R_8$ wie oben definiert ist, mit der Maßgabe, daß, wenn $R_1$ Wasserstoff oder $C_1$-$C_4$-Alkyl ist, $R_2$ von einer $C_1$-$C_4$-Alkylgruppe oder einer Cyclohexylgruppe unterschiedlich ist,
sowie deren Additionssalze mit pharmazeutisch akzeptablen Mineral- oder organischen Säuren.

**2.** Verbindungen nach Anspruch 1, dadurch gekennzeichnet, daß $R_1$ und $R_2$ jeweils unabhängig voneinander eine ein- oder mehrmals durch Chloratom, Fluoratom oder eine Methylgruppe substituierte Phenylgruppe darstellen.

**3.** Verbindungen nach einem der Ansprüche 1 oder 2, dadurch gekennzeichnet, daß $R_4$ die Gruppe

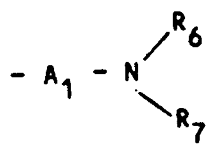

darstellt, worin $A_1$ eine gerade oder verweigte $C_2$-$C_5$-Alkylgruppe bezeichnet, $R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5- oder 6-gliedrigen Heterocyclus bilden, der gegebenenfalls ein zweites Heteroatom enthält, insbesondere die Pyrrolidin-, Pipiridin-, Morpholin- und N-Alkylpiperazinringe.

**4.** Verbindungen nach einem der Ansprüche 1 bis 3, dadurch gekennzeichnet, daß $R_4$ eine Gruppe

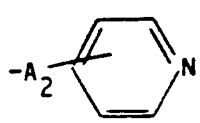

darstellt, worin $A_2$ eine Gruppe $(CH_2)_m$ mit m = 0, 1, 2, 3 bezeichnet, die den Pyridinring in der 2-, 3- oder 4-Stellung substituiert.

5. Verbindungen nach einem der Ansprüche 1 bis 3, dadurch gekennzeichnet, daß $R_4$ eine Gruppe

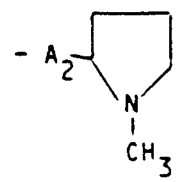

darstellt, worin $A_2$ wie oben definiert ist.

6. Verbindungen nach einem der Ansprüche 1 bis 5, dadurch gekennzeichnet, daß $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha$,$\alpha$-Dimethylbenzyl-; Cyclohexyl-; Diphenyl- oder Adamantylgruppe darstellt, mit der Maßgabe, daß, wenn eine der Gruppen $R_1$ oder $R_2$ Wasserstoff ist, die andere nicht H oder Methyl ist.

7. Verbindungen nach einem der Ansprüche 1 bis 5, dadurch gekennzeichnet, daß $R_1$ und $R_2$ zusammen eine Gruppe

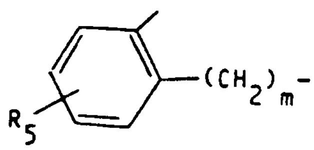

darstellen, worin die Phenylgruppe das Thiazol in der 4-Stellung und die Gruppe $(CH_2)_m$ in der 5-Stellung substituiert und worin m eine ganze Zahl gleich 2 oder 3 ist und $R_5$ Wasserstoff oder eine Nitrogruppe darstellt.

8. Thiazolderivat der Formel (I):

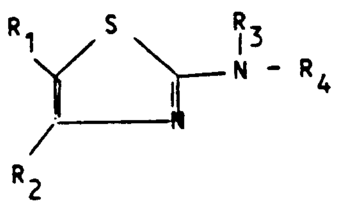

(I)

worin
$R_1$ eine Phenylgruppe ist,
$R_2$ eine Benzylgruppe ist,
$R_3$ Wasserstoff ist, und
$R_4$ die Gruppe $(CH_2)_3$-$N(C_2H_5)_2$ ist,
in Dihydrochloridform.

9. Verfahren zur Herstellung der Verbindungen der Formel I, dadurch gekennzeichnet, daß man in saurem Medium bei pH 1 bis 6 einen substiuierten Thioharnstoff der Formel

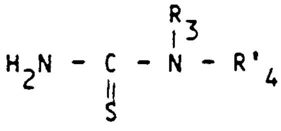

,

worin $R_3$ die obige Bedeutung hat und $R'_4$ dieselben Bedeutungen wie $R_4$ hat, ausgenommen für den Fall, daß $R_4$ ein primäres oder sekundäres bin enthält, worin $R'_4$ dann die $R_4$ entsprechende Gruppe ist, in welcher ein Wasserstoff der Aminfunktion durch eine gegen die Hydrolyse in alkalischem Medium beständige Schutzgruppe ersetzt worden ist, mit einem alpha-Bromcarbonylderivat der Formel

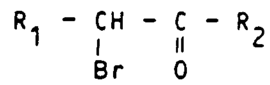

oder mit dem entsprechenden Bromderivat, in dem die Carbonylgruppe in der Acetalform geschützt ist, erhitzt, um die Verbindungen (I) zu erhalten, und gegebenenfalls die Verbindung I nach einem bekannten Verfahren in das Salz überführt.

**10.** Pharmazeutische Zusammensetzungen, die als Wirkstoff eine Verbindung der Formel (I) oder eines ihrer pharmazeutisch akzeptablen Salze nach einem der Ansprüche 1 bis 8 in Kombination mit einem pharmazeutisch akzeptablen Träger enthalten.

**11.** Pharmazeutische Zusammensetzungen nach Anspruch 10, die 20 bis 500 mg Wirkstoff enthalten.

**12.** Verwendung der Thiazolderivate der Formel

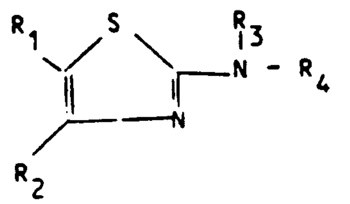

(I)

worin:

- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha$,$\alpha$-Dimethylbenzyl-; Cyclohexyl-; Diphenyl-; Thienyl- oder Adamantylgruppe darstellt; oder aber $R_1$ und $R_2$ zusammen eine Gruppe

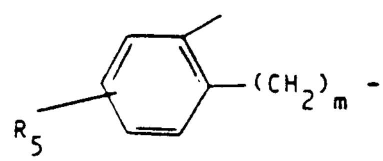

darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet;
mit der Maßgabe, daß, wenn eine der Gruppen $R_1$ oder $R_2$ Wasserstoff ist, die andere nicht H oder Methyl ist;
- $R_3$ Wasserstoff oder eine $C_1$-$C_4$-Alkylgruppe darstellt;

- $R_4$ darstellt: eine Gruppe

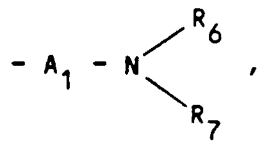

worin
$A_1$ eine gerade oder verzweigte $C_2$-$C_5$-Alkylgruppe bezeichnet,
$R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein zweites Heteroatom enthält, insbesondere die Pyrrolidin-, Piperidin-, Morpholin- und N-Alkylpiperazinringe;

- eine Gruppe

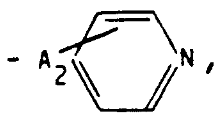

worin $A_2$ eine Gruppe $(CH_2)_m$ mit m = 0, 1, 2, 3 ist, die den Pyridinring in Stellung 2, 3 oder 4 substituiert;

- eine Gruppe

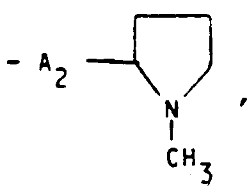

worin $A_2$ wie oben definiert ist;

- eine Gruppe

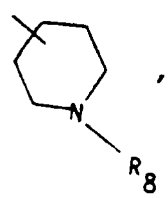

worin $R_8$ eine $C_1$-$C_4$-Alkylgruppe ist; oder aber der Substituent

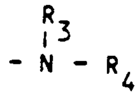

eine Gruppe

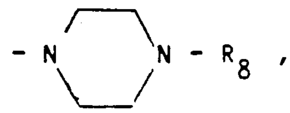

worin $R_8$ wie oben definiert ist, darstellt,
oder ihrer Additionssalze mit pharmazeutisch akzeptablen Mineral-oder organischen Säuren,
zur Herstellung von Medikamenten, die zur Behandlung von altersbedingten Degenerationserscheinungen nützlich sind.

**Patentansprüche für folgenden Vertragsstaat : ES**

1. Verfahren zur Herstellung von auf das cholinerge System wirkenden Thiazolderivaten der allgemeinen Formel

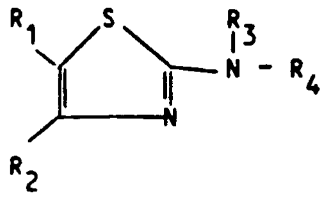

(I)

worin:
- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha,\alpha$-Dimethylbenzyl-; Cyclohexyl-; Diphenyl-; Thienyl- oder Adamantylgruppe darstellt; oder aber $R_1$ und $R_2$ zusammen eine Gruppe

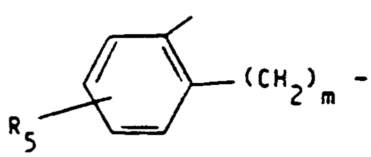

darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet;
mit der Maßgabe, daS, wenn eine der Gruppen $R_1$ oder $R_2$ Wasserstoff ist, die andere nicht H oder Methyl ist;
- $R_3$ Wasserstoff oder eine $C_1$-$C_4$-Alkylgruppe darstellt;
- $R_4$ darstellt:
  - eine Gruppe

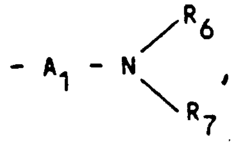

worin $A_1$ eine gerade oder verzweigte $C_2$-$C_5$-Alkylgruppe bezeichnet,
$R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein

zweites Heteroatom enthält, insbesondere die Pyrrolidin-, Piperidin-, Morpholin- und N-Alkylpiperazinringe;

- eine Gruppe

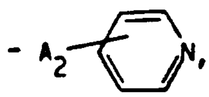

worin $A_2$ eine Gruppe $(CH_2)_m$ mit m = 0, 1, 2, 3 ist, die den Pyridinring in Stellung 2, 3 oder 4 substituiert;

- eine Gruppe

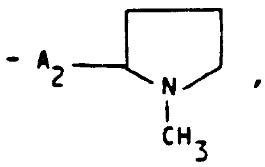

worin $A_2$ wie oben definiert ist;

- eine Gruppe

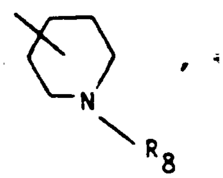

worin $R_8$ eine $C_1$-$C_4$-Alkylgruppe ist; oder aber der Substituent

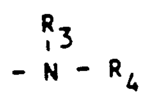

eine Gruppe

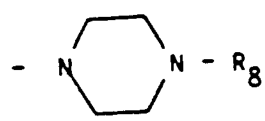

darstellt, worin $R_8$ wie oben definiert ist, mit der Maßgabe, daß, wenn $R_1$ Wasserstoff oder $C_1$-$C_4$-Alkyl ist, $R_2$ von einer $C_1$-$C_4$-Alkylgruppe oder einer Cyclohexylgruppe unterschiedlich ist,

sowie deren Additionssalze mit pharmazeutisch akzeptablen Mineral- oder organischen Säuren,

dadurch gekennzeichnet, daß man in saurem Medium bei pH 1 bis 6 einen substiuierten Thioharnstoff der Formel

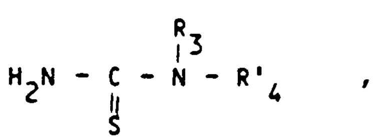

worin $R_3$ die obige Bedeutung hat und $R'_4$ dieselben Bedeutungen wie $R_4$ hat, ausgenommen für den Fall, daß $R_4$ ein primäres oder sekundäres Amin enthält, worin $R'_4$ dann die $R_4$ entsprechende Gruppe ist, in welcher ein Wasserstoff der Aminfunktion durch eine gegen die Hydrolyse in alkalischem

Medium beständige Schutzgruppe ersetzt worden ist, mit einem alpha-Bromcarbonylderivat der Formel

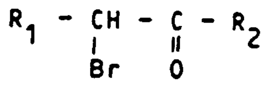

oder mit dem entsprechenden Bromderivat, in dem die Carbonylgruppe in der Acetalform geschützt ist, erhitzt, um die Verbindungen (I) zu erhalten, und gegebenenfalls die Verbindung I nach einem bekannten Verfahren in das Salz überführt.

**2.** Verfahren nach Anspruch 1, dadurch gekennzeichnet, daß man ein alpha-Bromcarbonylderivat verwendet, in dem $R_1$ und $R_2$ jeweils unabhängig voneinander eine ein- oder mehrmals durch Chloratom, Fluoratom oder eine Methylgruppe substituierte Phenylgruppe darstellen.

**3.** Verfahren nach einem der Ansprüche 1 oder 2, dadurch gekennzeichnet, daß $R'_4$ die Gruppe

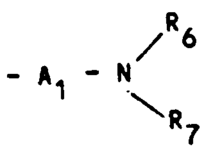

darstellt, worin $A_1$ eine gerade oder verweigte $C_2$-$C_5$-Alkylgruppe bezeichnet, $R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5- oder 6-gliedrigen Heterocyclus bilden, der gegebenenfalls ein zweites Heteroatom enthält, insbesondere die Pyrrolidin-, Pipiridin-, Morpholin- und N-Alkylpiperazinringe.

**4.** Verfahren nach einem der Ansprüche 1 oder 2, dadurch gekennzeichnet, daß $R'_4$ eine Gruppe

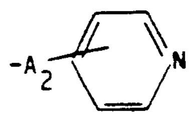

darstellt, worin $A_2$ eine Gruppe $(CH_2)_m$ mit m = 0, 1, 2, 3 bezeichnet, die den Pyridinring in der 2-, 3- oder 4-Stellung substituiert.

**5.** Verfahren nach einem der Ansprüche 1 oder 2, dadurch gekennzeichnet, daß $R'_4$ eine Gruppe

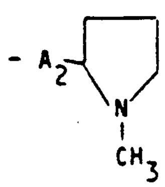

darstellt, worin $A_2$ wie in Anspruch 1 definiert ist.

**6.** Verfahren nach einem der Ansprüche 1 bis 5, dadurch gekennzeichnet, daß $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha,\alpha$-Dimethylbenzyl-; Cyclohexyl-; Diphenyl- oder Adamantylgruppe darstellt, mit der Maßgabe, daß, wenn eine der Gruppen $R_1$ oder $R_2$ Wasserstoff ist, die andere nicht H oder Methyl ist.

**7.** Verfahren nach einem der Ansprüche 1 bis 5, dadurch gekennzeichnet, daß $R_1$ und $R_2$ zusammen eine Gruppe

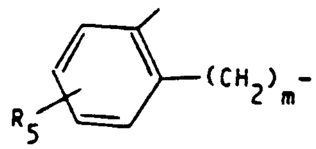

darstellen, worin die Phenylgruppe das Thiazol in der 4-Stellung und die Gruppe $(CH_2)_m$ in der 5-Stellung substituiert und worin m eine ganze Zahl gleich 2 oder 3 ist und $R_5$ Wasserstoff oder eine Nitrogruppe darstellt.

**8.** Verfahren zur Herstellung des Thiazolderivats der Formel (I):

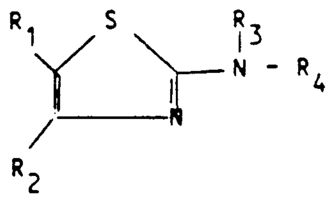

(I)

worin

$R_1$ eine Phenylgruppe ist,
$R_2$ eine Benzylgruppe ist,
$R_3$ Wasserstoff ist, und
$R_4$ die Gruppe $(CH_2)_3$-$N(C_2H_5)_2$ ist,

in Dihydrochloridform, dadurch gekennzeichnet, daß es darin besteht, daß man in saurem Medium bei pH 1 bis 6 einen substituierten Thioharnstoff der Formel

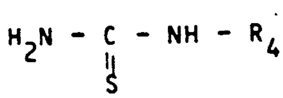

worin $R_4$ die Gruppe $-(CH_2)_3$-$N(C_2H_5)_2$ ist, mit dem alpha-Bromcarbonylderivat der Formel:

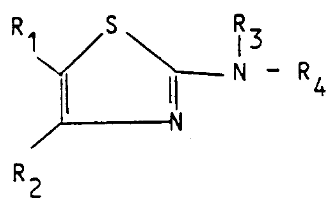

(I)

erhitzt und die so erhaltene Verbindung in das Dihydrochlorid übergeführt wird.

**9.** Verwendung der Thiazolderivate der Formel:

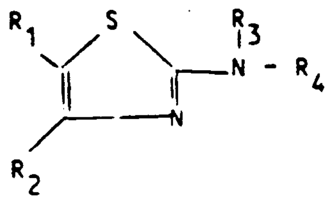

(I)

worin:

- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; α,α-Dimethylbenzyl-; Cyclohexyl-; Diphenyl-; Thienyl- oder Adamantylgruppe darstellt; oder aber $R_1$ und $R_2$ zusammen eine Gruppe

  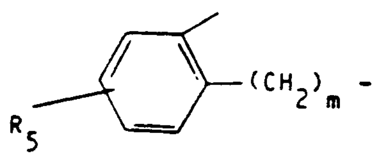

  darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet;
  mit der Maßgabe, daß, wenn eine der Gruppen $R_1$ oder $R_2$ Wasserstoff ist, die andere nicht H oder Methyl ist;
- $R_3$ Wasserstoff oder eine $C_1$-$C_4$-Alkylgruppe darstellt;
- $R_4$ darstellt: eine Gruppe

  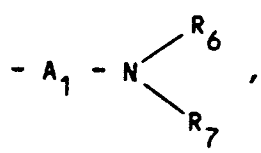

  worin
  $A_1$ eine gerade oder verzweigte $C_2$-$C_5$-Alkylgruppe bezeichnet,
  $R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein zweites Heteroatom enthält, insbesondere die Pyrrolidin-, Piperidin-, Morpholin- und N-Alkylpiperazinringe;
  - eine Gruppe

    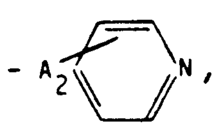

    worin $A_2$ eine Gruppe $(CH_2)_m$ mit m = 0, 1, 2, 3 ist, die den Pyridinring in Stellung 2, 3 oder 4 substituiert;

- eine Gruppe

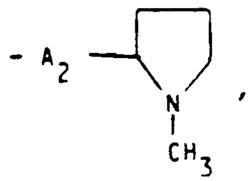

worin $A_2$ wie oben definiert ist;

- eine Gruppe

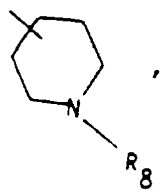

worin $R_8$ eine $C_1$-$C_4$-Alkylgruppe ist; oder aber der Substituent

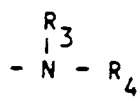

eine Gruppe

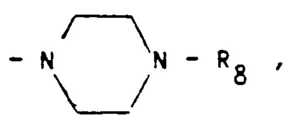

worin $R_8$ wie oben definiert ist, darstellt, oder ihrer Additionssalze mit pharmazeutisch akzeptablen Mineral-oder organischen Säuren,
zur Herstellung von Medikamenten, die zur Behandlung von altersbedingten Degenerationserscheinungen nützlich sind.

**Patentansprüche für folgenden Vertragsstaat : GR**

**1.** Neue Thiazolderivate der allgemeinen Formel

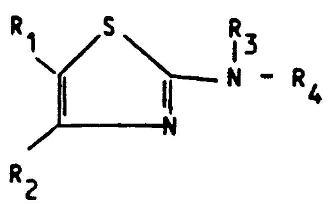

(I)

worin:

- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine

Naphthyl-; Benzyl-; α,α-Dimethylbenzyl-; Cyclohexyl-; Diphenyl-; Thienyl- oder Adamantylgruppe darstellt; oder aber $R_1$ und $R_2$ zusammen eine Gruppe

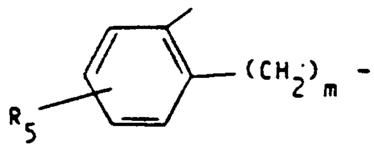

darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet;
mit der Maßgabe, daß, wenn eine der Gruppen $R_1$ oder $R_2$ Wasserstoff ist, die andere nicht H oder Methyl ist;

- $R_3$ Wasserstoff oder eine $C_1$-$C_4$-Alkylgruppe darstellt;
- $R_4$ darstellt:
  - eine Gruppe

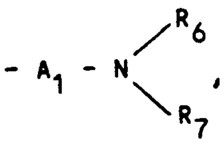

worin
$A_1$ eine gerade oder verzweigte $C_2$-$C_5$-Alkylgruppe bezeichnet,
$R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein zweites Heteroatom enthält, insbesondere die Pyrrolidin-, Piperidin-, Morpholin- und N-Alkylpiperazinringe;

  - eine Gruppe

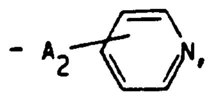

worin $A_2$ eine Gruppe $(CH_2)_m$ mit m = 0, 1, 2, 3 ist, die den Pyridinring in Stellung 2, 3 oder 4 substituiert;

  - eine Gruppe

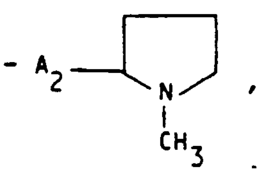

worin $A_2$ wie oben definiert ist;

- eine Gruppe

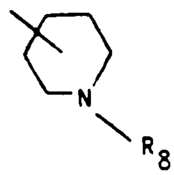

worin $R_8$ eine $C_1$-$C_4$-Alkylgruppe ist; oder aber der Substituent

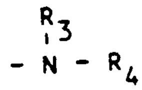

eine Gruppe

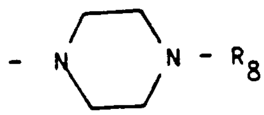

darstellt, worin $R_8$ wie oben definiert ist, mit der Maßgabe, daß, wenn $R_1$ Wasserstoff oder $C_1$-$C_4$-Alkyl ist, $R_2$ von einer $C_1$-$C_4$-Alkylgruppe oder einer Cyclohexylgruppe unterschiedlich ist,

sowie deren Additionssalze mit pharmazeutisch akzeptablen Mineral- oder organischen Säuren.

**2.** Verbindungen nach Anspruch 1, dadurch gekennzeichnet, daß $R_1$ und $R_2$ jeweils unabhängig voneinander eine ein- oder mehrmals durch Chloratom, Fluoratom oder eine Methylgruppe substituierte Phenylgruppe darstellen.

**3.** Verbindungen nach einem der Ansprüche 1 oder 2, dadurch gekennzeichnet, daß $R_4$ die Gruppe

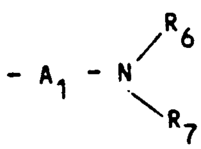

darstellt, worin $A_1$ eine gerade oder verweigte $C_2$-$C_5$-Alkylgruppe bezeichnet, $R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5- oder 6-gliedrigen Heterocyclus bilden, der gegebenenfalls ein zweites Heteroatom enthält, insbesondere die Pyrrolidin-, Pipiridin-, Morpholin- und N-Alkylpiperazinringe.

**4.** Verbindungen nach einem der Ansprüche 1 bis 3, dadurch gekennzeichnet, daß $R_4$ eine Gruppe

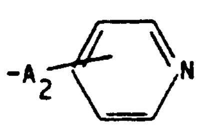

darstellt, worin $A_2$ eine Gruppe $(CH_2)_m$ mit m = 0, 1, 2, 3 bezeichnet, die den Pyridinring in der 2-, 3- oder 4-Stellung substituiert.

5. Verbindungen nach einem der Ansprüche 1 bis 3, dadurch gekennzeichnet, daß $R_4$ eine Gruppe

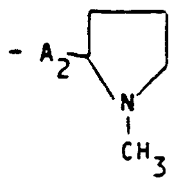

darstellt, worin $A_2$ wie oben definiert ist.

6. Verbindungen nach einem der Ansprüche 1 bis 5, dadurch gekennzeichnet, daß $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha,\alpha$-Dimethylbenzyl-; Cyclohexyl-; Diphenyl- oder Adamantylgruppe darstellt, mit der Maßgabe, daß, wenn eine der Gruppen $R_1$ oder $R_2$ Wasserstoff ist, die andere nicht H oder Methyl ist.

7. Verbindungen nach einem der Ansprüche 1 bis 5, dadurch gekennzeichnet, daß $R_1$ und $R_2$ zusammen eine Gruppe

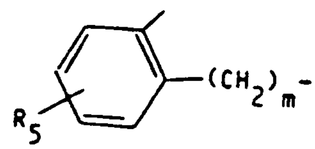

darstellen, worin die Phenylgruppe das Thiazol in der 4-Stellung und die Gruppe $(CH_2)_m$ in der 5-Stellung substituiert und worin m eine ganze Zahl gleich 2 oder 3 ist und $R_5$ Wasserstoff oder eine Nitrogruppe darstellt.

8. Thiazolderivat der Formel (I):

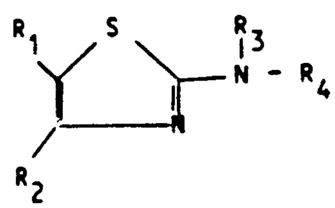

(I)

worin
$R_1$ eine Phenylgruppe ist,
$R_2$ eine Benzylgruppe ist,
$R_3$ Wasserstoff ist, und
$R_4$ die Gruppe $(CH_2)_3$-$N(C_2H_5)_2$ ist,
in Dihydrochloridform.

9. Verfahren zur Herstellung der Verbindungen der Formel I, dadurch gekennzeichnet, daß man in saurem Medium bei pH 1 bis 6 einen substiuierten Thioharnstoff der Formel

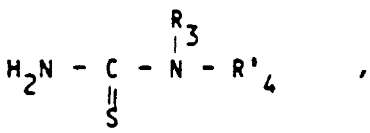

worin $R_3$ die obige Bedeutung hat und $R'_4$ dieselben Bedeutungen wie $R_4$ hat, ausgenommen für den Fall, daß $R_4$ ein primäres oder sekundäres bin enthält, worin $R'_4$ dann die $R_4$ entsprechende Gruppe ist, in welcher ein Wasserstoff der Aminfunktion durch eine gegen die Hydrolyse in alkalischem Medium beständige Schutzgruppe ersetzt worden ist, mit einem alpha-Bromcarbonylderivat der Formel

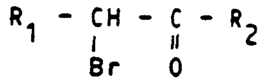

oder mit dem entsprechenden Bromderivat, in dem die Carbonylgruppe in der Acetalform geschützt ist, erhitzt, um die Verbindungen (I) zu erhalten, und gegebenenfalls die Verbindung I nach einem bekannten Verfahren in das Salz überführt.

**10.** Verwendung der Thiazolderivate der Formel

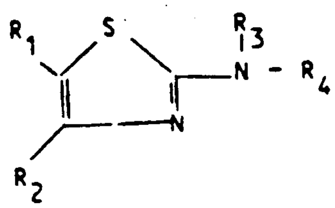

(I)

worin:

- $R_1$ und $R_2$ jeweils unabhängig voneinander Wasserstoff; eine $C_1$-$C_4$-Alkylgruppe; eine Phenylgruppe; eine durch ein Halogenatom oder eine $C_1$-$C_4$-Alkylgruppe oder eine $C_1$-$C_4$-Alkoxygruppe, eine Nitrogruppe oder eine Hydroxygruppe ein- oder mehrmals substituierte Phenylgruppe darstellt; oder eine der Gruppen $R_1$ und $R_2$ Wasserstoff bezeichnet und die andere eine Naphthyl-; Benzyl-; $\alpha,\alpha$-Dimethylbenzyl-; Cyclohexyl-; Diphenyl-; Thienyl- oder Adamantylgruppe darstellt; oder aber $R_1$ und $R_2$ zusammen eine Gruppe

  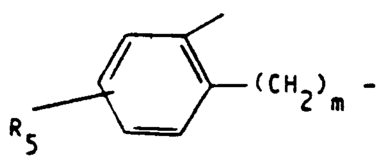

  darstellen, worin die Phenylgruppe in der 4-Stellung des Thiazols und die Gruppe $(CH_2)_m$ in der 5-Stellung gebunden ist, und worin m eine ganze Zahl gleich 2 oder 3 ist und $R_5$ Wasserstoff oder eine Nitrogruppe, die eine der freien Stellungen des Ringes einnimmt, bezeichnet;
  mit der Maßgabe, daß, wenn eine der Gruppen $R_1$ oder $R_2$ Wasserstoff ist, die andere nicht H oder Methyl ist;
- $R_3$ Wasserstoff oder eine $C_1$-$C_4$-Alkylgruppe darstellt;
- $R_4$ darstellt: eine Gruppe

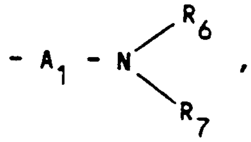

worin $A_1$ eine gerade oder verzweigte $C_2$-$C_5$-Alkylgruppe bezeichnet,
$R_6$ und $R_7$ unabhängig voneinander Wasserstoff, eine $C_1$-$C_4$-Alkylgruppe oder eine $C_3$-$C_6$-Cycloalkylgruppe darstellen, oder aber $R_6$ und $R_7$ zusammen mit dem Stickstoffatom, an das sie gebunden sind, einen 5-oder 6-gliedrigen Heterocyclus darstellen, der gegebenenfalls ein zweites Heteroatom enthält, insbesondere die Pyrrolidin-, Piperidin-, Morpholin- und N-Alkylpiperazinringe;

- eine Gruppe

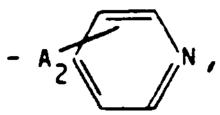

worin $A_2$ eine Gruppe $(CH_2)_m$ mit m = 0, 1, 2, 3 ist, die den Pyridinring in Stellung 2, 3 oder 4 substituiert;

- eine Gruppe

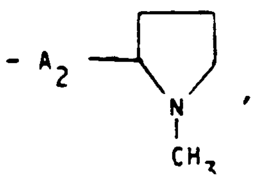

worin $A_2$ wie oben definiert ist;

- eine Gruppe

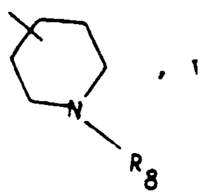

worin $R_8$ eine $C_1$-$C_4$-Alkylgruppe ist; oder aber der Substituent

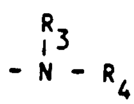

eine Gruppe

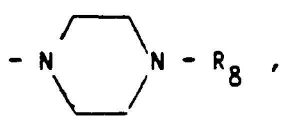

worin $R_8$ wie oben definiert ist, darstellt, oder ihrer Additionssalze mit pharmazeutisch akzeptablen Mineral-oder organischen Säuren,
zur Herstellung von Medikamenten, die zur Behandlung von altersbedingten Degenerationserscheinungen nützlich sind.